(19) Europäisches Patentamt
European Patent Office
Office européen des brevets

(11) EP 0 307 342 B1

(12) EUROPÄISCHE PATENTSCHRIFT

(45) Veröffentlichungstag und Bekanntmachung des Hinweises auf die Patenterteilung:
**03.01.1996 Patentblatt 1996/01**

(51) Int. Cl.[6]: **C07D 405/06**, C07D 213/55, C07D 239/26, C07D 405/14, A61K 31/365, A61K 31/44, A61K 31/505

(21) Anmeldenummer: **88710018.8**

(22) Anmeldetag: **07.07.1988**

(54) **3-Desmethyl-mevalonsäurederivate, Verfahren zu ihrer Herstellung, pharmazeutische Präparate auf Basis dieser Verbindungen, ihre Verwendung sowie Zwischenprodukte**

3-Demethyl-4-fluoro-mevalonic-acid derivatives, process for their preparation, pharmaceutical preparations based on those compounds, their use and intermediates

Dérivés d'acide 3-déméthyl-4-fluoro-mévalonique, procédé pour leur préparation, préparations pharmaceutiques à base de ces composés, leur utilisation et intermédiaires

(84) Benannte Vertragsstaaten:
**AT BE CH DE ES FR GB GR IT LI LU NL SE**

(30) Priorität: **10.07.1987 DE 3722808**

(43) Veröffentlichungstag der Anmeldung:
**15.03.1989 Patentblatt 1989/11**

(73) Patentinhaber: **HOECHST AKTIENGESELLSCHAFT**
**D-65926 Frankfurt am Main (DE)**

(72) Erfinder:
- **Kesseler, Kurt, Dr.**
  **D-6232 Bad Soden am Taunus (DE)**
- **Beck, Gerhard, Dr.**
  **D-6000 Frankfurt am Main (DE)**
- **Bartmann, Wilhelm, Prof. Dr.**
  **D-6232 Bad Soden am Taunus (DE)**
- **Granzer, Ernold, Dr.**
  **D-6233 Kelkheim (Taunus) (DE)**

(56) Entgegenhaltungen:
**EP-A- 0 194 093**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 307 342 B1

Printed by Rank Xerox (UK) Business Services
2.8/3.4

## Beschreibung

Derivate der 3-Hydroxy-3-methyl-glutarsäure (HMG) und der Mevalonsäure sind als Hemmer der Cholesterinbiosynthese beschrieben worden (M. T. Boots et al., J. Pharm. Sci. 69, 306 (1980), F. M. Singer et al., Proc. Soc. Exper. Biol. Med. 102, 270 (1959), H. Feres, Tetrahedron Lett. 24, 3769 (1983)). 3-Hydroxy-3-methyl-glutarsäure selbst zeigt an der Ratte und im Humanversuch signifikante cholesterinsenkende Wirkung (Z. Beg, Experimentia 23, 380 (1967), ibid 24, 15 (1968), P. J. Lupienet al., Lancet 1978, 1, 283)).

Endo et al. (Febs. Letter 72, 323 (1976), J. Biol. Chem. 253, 1121 (1978)) berichteten über die Hemmung der 3-Hydroxy-3-methyl-glutaryl-coenzym-A-Reduktase (HMG-CoA-Reduktase), des geschwindigkeitsbestimmenden Enzyms der Cholesterinbiosynthese, durch das Fermentationsprodukt "Compactin". Brown et al. (J. Chem. Soc. 1165 (1976)) bestimmten die chemische Struktur und die absolute Konfiguration des "Compactins" durch eine Kombination chemischer, spektroskopischer und radiokristallographischer Methoden und konnten zeigen, daß "Compactin" ein Derivat des 3-Desmethylmevalonsäurelactons ist.

Compactin-Derivate, die die Aktivität der HMG-CoA-Reduktase hemmen, wurden bereits beschrieben (G.E. Stokker et al., J. Med. Chem. 28, 347-358 (1985)).

Die vorliegende Erfindung betrifft neue synthetische Analoga des "Compactins" in Form des δ-Lacton der Formel oder in Form des Dihydroxysäure-Derivates II

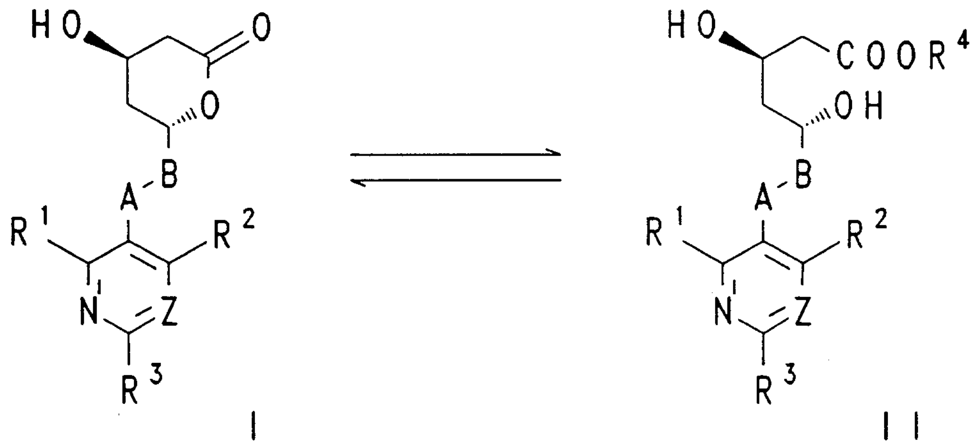

I II

In den Formeln bedeuten

| | |
|---|---|
| A-B | einen Rest der Formel -CH = CH- oder $-CH_2-CH_2-$ |
| Z | einen Rest der Formel -CH oder ein Stickstoffatom" |
| $R^1$, $R^2$, $R^3$ | unabhängig voneinander einen verzweigten Alkylrest mit bis zu 4 C-Atomen, einen Cycloalkylrest mit 3-6 C-Atomen oder einen Phenylrest, der gegebenenfalls 1 bis 2 gleich oder verschiedene Substituenten ausgewählt aus der Gruppe Fluor, Hydroxy und $C_1$-$C_4$-Alkyl trägt, |
| $R^4$ | Wasserstoff, Methyl, Ethyl, Isopropyl, Isobutyl, Benzyl, Natrium, Kalium, Ammonium ($NH_4$) oder Methyltris (hydroxymethyl)ammonium. |

Die Erfindung betrifft die reinen Enantiomeren mit der in der allgemeinen Formel I angegebenen absoluten Konfiguration 4R, 6S bzw. der in Formel II wiedergegebenen absoluten Konfiguration 3R, 5S.

Unter den Substituenten $R^1$ sind besonders bevorzugt: Isopropyl, sek.-Butyl, tert.-Butyl, Cyclopropyl, Cyclohexyl, Phenyl, 4-Chlorphenyl, 4-Fluorphenyl, 4-Hydroxyphenyl, 4-Methoxyphenyl, 4-Fluor-3-methylphenyl, 3,5-Dimethylphenyl, Cyclohexylmethyl, 4-Trifluormethylphenyl.

Unter den Substituenten $R^2$ sind besonders bevorzugt Isopropyl, sek.-Butyl, tert.-Butyl, Cyclopropyl, Cyclohexyl, Phenyl, 4-Chlorphenyl, 4-Fluorphenyl, 4-Hydroxyphenyl, 4-Methoxyphenyl, 4- Fluor-3-methylphenyl, 3,5-Dimethylphenyl, Cyclohexylmethyl, 4- Trifluormethylphenyl.

Unter den Substituenten $R^3$ sind besonders bevorzugt Isopropyl, tert.-Butyl, Cyclohexyl, Phenyl, 4-Flurophenyl, 4-Hydroxyphenyl, 2,5-Dimethylphenyl, 3,5-Dimethylphenyl, 4-Trifluormethylphenyl.

Unter den Substituenten $R^4$ sind besonders bevorzugt Wasserstoff, Methyl, Ethyl, Natrium, Kalium.

Ganz besonders bevorzugt sind Verbindungen der Formel 2, worin Z einen Rest der Formel -CH oder N, $R^1$ Isopropyl, Cyclopropyl, $R^2$ 4-Fluorphenyl, 4-Hydroxyphenyl, $R^3$ Isopropyl, tert.-Butyl, Cyclohexyl, Phenyl, 4-Hydroxyphenyl oder 4-Fluorphenyl bedeuten sowie die Natrium- und Kaliumsalze der entsprechenden Dihydroxycarbonsäuren der Formel II.

Die Erfindung betrifft ferner ein Verfahren zur Herstellung von Verbindungen der Formeln I und II, das dadurch gekennzeichnet ist, daß man

a) die Phosphoniumsalze der Formel III,

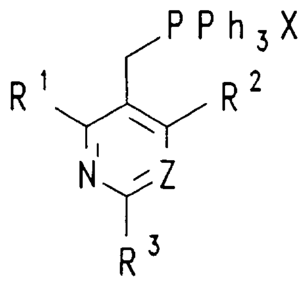

III

worin $R^1$, $R^2$, $R^3$ und Z die zur Formel I angegebene Bedeutung haben und X = Cl, Br, J ist, mit dem chiralen Aldehyd der Formel IV umsetzt,

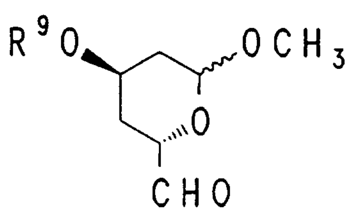

IV

worin $R^9$ eine gegen Basen und schwache Säuren stabile Schutzgruppe bedeutet, z.B. die $t\text{-}C_4H_9\,(C_6H_5)_2$ Si-Gruppe, zu einer Verbindung der Formel V

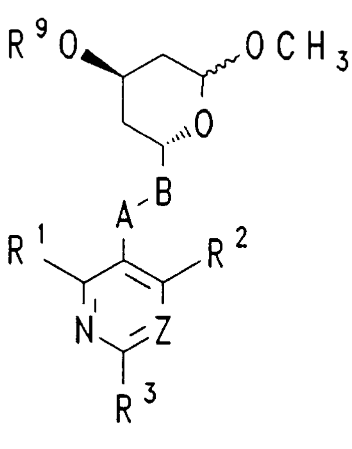

V

worin $R^1$, $R^2$, $R^3$ und Z die zur Formel I, $R^9$ die zur Formel IV gegebene Bedeutung haben und A-B die (-CH = CH-)-Gruppe darstellt,

b) in einer Verbindung der allgemeinen Formel V die Methylacetalfunktion sauer hydrolisiert zu einem Lactol der Formel VI

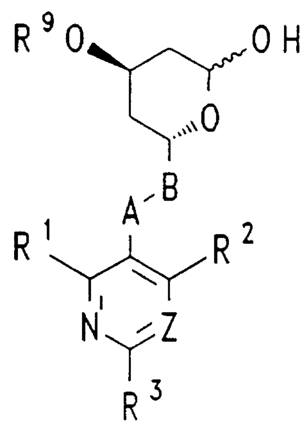

VI

worin $R^1$, $R^2$, $R^3$ und Z die zur Formel I, $R^9$ die zur Formel IV gegebene Bedeutung haben und A-B die (-CH = CH-)-Gruppe darstellt,

c) die Verbindung der Formel VI oxidiert zu einem Lacton der allgemeinen Formel VII,

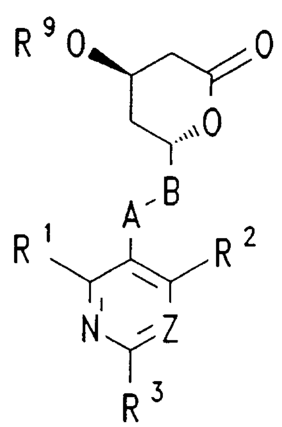

VII

worin $R^1$, $R^2$, $R^3$ und Z die zur Formel I, $R^9$ die zur Formel IV gegebene Bedeutung haben und A-B die (-CH = CH-)-Gruppe darstellt,

d) in einer Verbindung der allgemeinen Formel VII die Schutzgruppe $R^9$ abspaltet zu einer Verbindung der Formel I, worin $R^1$, $R^2$, $R^3$ und Z die zur Formel I angegebene Bedeutung haben und A-B die (-CH = CH-)-Gruppe darstellt,

e) gegebenenfalls eine erhaltene Verbindung der allgemeinen Formel I, worin A-B eine (-CH = CH-)-Gruppe darstellt, hydriert zu einer Verbindung der allgemeinen Formel I, in der A-B eine ($-CH_2-CH_2-$)-Gruppe darstellt, wobei die Hydrierung auch bei den Verbindungen der Formel V, VI oder VII erfolgen kann zu Verbindungen, worin A-B die ($-CH_2-CH_2-$)-Gruppe darstellt,

f) gegebenenfalls ein Hydroxylacton der allgemeinen Formel I in die entsprechende Dihydroxysäure der Formel II, bzw. deren Salze überführt oder gegebenenfalls aus dem Hydroxylacton I oder der freien Hydroxysäure II die entsprechenden Ester darstellt.

Die im erfindungsgemäßen Verfahren als Ausgangsmaterial verwandten Phosphoniumsalze der allgemeinen Formel III, worin $R^1$, $R^2$ und $R^3$ die zur allgemeinen Formel I gegebene Bedeutung haben, erhält man wie im Schema 1 dargestellt.

Ketone der allgemeinen Formel VIII, wobei $R^2$ und $R^3$ die angegebene Bedeutung haben, sind literaturbekannt oder können nach literaturbekannten Verfahren hergestellt werden (vgl. z.B. D. Vorländer und F. Kalkow, Berichte d. Dtsch.

Chem. Ges. 30, 2268 (1897) oder H. Stetter in Houben-Weyl, Methoden der Organischen Chemie Bd. VII/26, 1449-1507, Thieme, Stuttgart 1976). Ebenfalls literaturbekannt oder nach literaturbekannten Verfahren darstellbar (z.B. analog M. Jackman, M. Klenk, B. Fishburn, B.F. Tullar und S. Archer, J. Am. Chem. Soc. 70, 2884 (1948)) sind die β-Ketoester der allgemeinen Formel IX, wobei $R^1$ die oben angegebene Bedeutung hat und $R^{10}$ einen geradkettigen oder verzweigten Alkylrest mit bis zu 6 C-Atomen bedeutet, vorzugsweise einen Methyl- oder Ethylrest.

Die Darstellung von Verbindungen der Formel X, worin $R^1$, $R^2$, $R^3$ und $R^{10}$ die angegebene Bedeutung haben, erfolgt analog Literaturverfahren, z.B. nach R. Connor, D.B. Andrews, J. Am. Chem. Soc. 56, 2713 (1943) und dort zitierte Literatur. Zur Überführung von Verbindungen des Typs X in Pyridine der allgemeinen Formel XV ($R^1$, $R^2$, $R^3$ haben hierbei die oben angegebene Bedeutung und Z bedeutet eine CH-Gruppe) arbeitet man z.B. nach einem Verfahren, das von F. Rehberg und F. Kröhnke, Liebigs Ann. Chem. 717, 91 (1968) beschrieben wurde.

Dihydropyrimidine der allgemeinen Formel XIV sind z.B. analog einem Literaturverfahren (E.F. Silversmith, J. Org. Chem. 27, 4090 (1962)) darstellbar, oder z.B. auch durch eine Synthese gemäß Schema 1, Weg A, indem man einen β-Ketoester der allgemeinen Formel IX mit einem Aldehyd des Typs XI zu einer Verbindung der allgemeinen Formel XII und diese ohne weitere Reinigung mit einer Amidiniumverbindung des Typs XIII zu einem Dihydropyrimidincarbonsäureester der allgemeinen Formel XIV umsetzt. Die Darstellung von Verbindungen des Typs XIV aus Komponenten der allgemeinen Formeln IX, XI und XIII kann ebenso als Eintopfreaktion durchgeführt werden (Schema 1, Weg B).

Die Oxidation von Verbindungen der Formel XIV zu Pyrimidincarbonsäureestern der allgemeinen Formel XV, worin $R^1$, $R^2$, $R^3$ und $R^{10}$ die oben angegebene Bedeutung haben und Z ein Stickstoffatom bedeutet, erfolgt analog literaturbekannten Verfahren, z.B. durch Dehydrierung mit Chloranil oder 2,3-Dichlor-5,6-dicyan-p-benzochinon (DDQ), wie von E.A. Braude, J. Hannah, R. Linstead, J. Chem. Soc. 1960, 3257 beschrieben.

Die Reduktion von Verbindungen der allgemeinen Formel XV erfolgt durch Umsetzung mit komplexen Metallhydriden, wie z.B. Lithiumaluminiumhydrid oder Diisobutylaluminiumhydrid in aprotischen Lösungsmitteln, z.B. Diethylether oder Tetrahydrofuran, bei Temperaturen zwischen -30°C und + 50°C.

Alkylhalogenide der allgemeinen Formel XVII, wobei $R^2$, $R^2$, $R^3$ und X die oben angegebene Bedeutung haben, lassen sich aus Alkoholen des Typs XVI z.B. durch Umsetzung mit Phosphorhalogeniden in inerten Lösungsmitteln wie z.B. Dichlormethan oder Toluol bei Temperaturen zwischen 0 und 100°C oder durch Umsetzung mit Halogenwasserstoffsäuren darstellen.

Phosphoniumsalze der allgemeinen Formel III erhält man z.B. durch Umsetzung der Alkylhalogenide XVII mit Triphenylphosphan in inerten Lösungsmitteln wie Toluol bei Temperaturen zwischen 20°C und 120°C (vgl. Schema I).

SCHEMA I

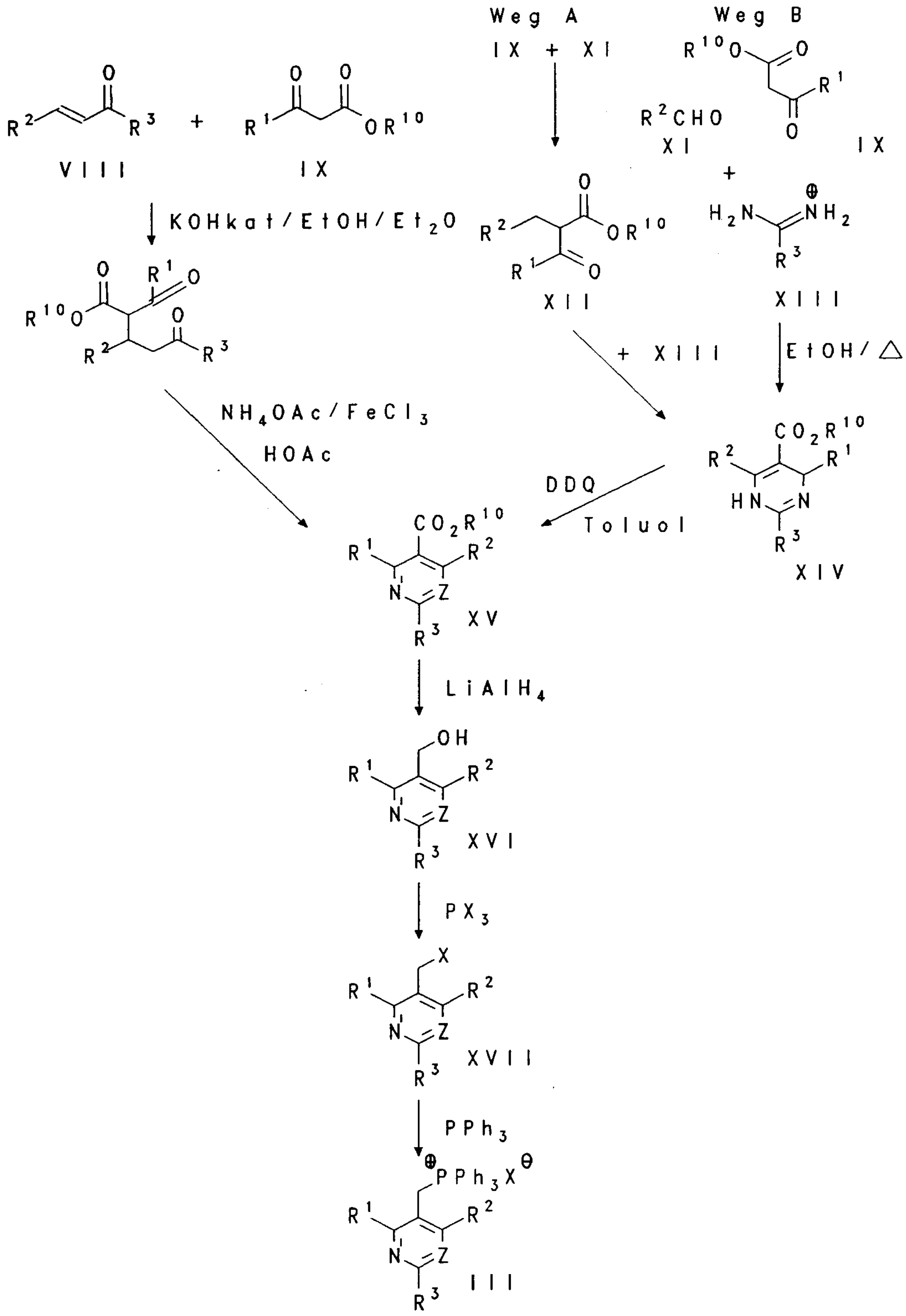


VIII
IX
XI
IX
XII
XIII
XIV
XV
XVI
XVII
III

Den im erfindungsgemäßen Verfahren als Ausgangsmaterial verwandten chiralen Aldehyd der Formel IV erhält man nach einem literaturbekannten Verfahren (Yuh Lin, J. R. Falck, Tetrahedron Letters 23, 4305-4308 (1982) aus dem entsprechenden Alkohol durch Oxidation mit z.B. $CrO_3$ oder Oxalchlorid/Dimethylsulfoxid in Gegenwart von Triethylamin.

Die Umsetzung des chiralen Aldehyds der Formel IV mit einem Phosphoniumsalz der Formel III nach Wittig (z.B. Wittig, Haag, Chem. Ber. 88, 1654 (1955)) ergibt die Verbindungen der Formel V, wobei eine bevorzugte Ausführungsform darin besteht, daß man die Phosphoniumsalze der Formel III in einem Lösungsmittel wie Tetrahydrofuran, Dimethylsulfoxid, DME, löst bzw. suspendiert und mit einer geeigneten starken Base wie z.B. Natriumhydrid, Kalium-tert. butylat, Li-äthylat oder Butyllithium die entsprechenden Phosphorane freisetzt und anschließend den Aldehyd der Formel IV zugibt und bei -10°C bis + 50°C 1-6 Std. reagieren läßt.

Die Verbindungen der Formel V werden dabei überwiegend in Form der Mischung der E/Z-Olefine erhalten. Mischungen von E/Z-Olefinen können gegebenenfalls chromatographisch aufgetrennt werden. Die reinen Z-Olefine können auch, wie bei G. Drefahl Chem. Ber. 94, 907 (1961) beschrieben durch Belichten der E/Z-Mischung in Lösungen, wie z.B. Toluol, Nitrobenzol, erhalten werden.

Die entsprechenden reinen E-Olefine können wie von De Tar et. al. in J. Amer. Chem. Soc. 78, 474 (1955) beschrieben durch Erwärmen der E/Z-Mischungen in Lösung in Gegenwart von Jod erhalten werden.

In den Verbindungen der Formel V läßt sich die Methylacetalschutzgruppe in der allgemein üblichen Weise durch saure Hydrolyse selektiv abspalten, bevorzugt mit einer Mischung von Eisessig, Tetrahydrofuran, Wasser im Verhältnis 3:2:2 bei + 20° bis + 90°C innerhalb von 6-24 Stunden.

Die Oxydation der Verbindungen der Formel VI zu einem Lakton der Formel VII kann durch Oxydationsmittel wie $CrO_3$ x 2Pyr, Pyridiumchlorochromat in inerten Lösungsmitteln, wie z.B. Methylenchlorid oder Chloroform erfolgen. Weitere Möglichkeiten der Oxydation bestehen in der Reaktion mit Thioanisol/$Cl_2$/$NEt_3$ in Tetrachlorkohlenstoff, in der Umsetzung mit DMSO/Oxalylchlorid/$NEt_3$ bei -20°C oder in der Umsetzung mit N-Jodsuccinimid/Tetrabutylammoniumjodid in Dichlormethan.

Zur Darstellung der Verbindungen der Formel I wird die Schutzgruppe $R^9$ in den Verbindungen der Formel VII abgespalten. Dies kann mit starken Säuren wie 5 normaler Salzsäure oder Schwefelsäure bei -10°C bis +30°C geschehen, oder mit Fluoridionen, bevorzugt durch Lösen der Verbindungen der Formel VII in Tetrahydrofuran oder Diethylether und Zugabe einer Mischung von Tetrabutylammoniumfluorid und Eisessig mit anschließendem Rühren bei 0°C bis 40°C zwischen 1 bis 12 Stunden.

Verbindungen der Formel I, bei denen A-B eine (CH = CH)-Gruppe darstellt, werden nach allgemein üblicher Methode, zweckmäßig bei Temp. zwischen 20°C und 40°C mit Wasserstoff in Gegenwart eines Metall-Katalysators, bevorzugt Palladium, Platin, $PtO_2$ oder $PdO_2$ zu Verbindungen der Formel I, bei denen A-B eine -CH2-CH2-Gruppe bedeutet, hydriert. Dabei kann bei Normaldruck in üblichen Lösungsmitteln wie Tetrahydrofuran, Essigester, Methanol, niedermolekularen Alkohlen, Eisessig, Chloroform hydriert werden, oder in Autoklaven bei erhöhtem Druck (2-50 atm). Die Hydrierung der -CH = CH-Gruppe kann auch bei den Verbindungen der Formeln V, VI oder VII erfolgen.

Die resultierenden Verbindungen der Formel I können in einfacher Weise durch Eindampfen des Lösungsmittels isoliert werden, gegebenenfalls nach chromatographischer Reinigung.

Die Verbindungen der Formel I fallen in optisch reiner Form an. Bezüglich der Konfiguration der Doppelbindung (A-B) = -CH = CH- erhält man E/Z-Gemische, diese lassen sich auf allen Synthesestufen chromatographisch auftrennen, oder zur E-Form isomerisieren (vgl. dazu De Tar et al. J. Amer. Chem. Soc. 78 475 (1955)).

Verbindungen der Formel I, in Form des δ-Lactons können in alkalischem Medium zu den entsprechenden Salzen der Dihydroxysäuren verseift werden, z.B. mit NaOH oder KOH in einem niedermolekularen Alkohol wie Methanol oder in Ethern wie Dimethoxyethan oder THF, gegebenenfalls in Gegenwart von Wasser. In den erhaltenen Salzen der Dihydroxysäuren läßt sich das Alkalikation nach Ansäuern in Ionenaustauschern in üblicher Weise gegen beliebige Kationen austauschen. Dazu läßt man z.B. die Dihydroxysäuren durch eine mit einem Kationenaustauscher, wie z.B. auf Basis Polystyrol/Divinylbenzol (®Amberlite CG-150 oder ®Dowex-CCR-2) gefüllte Säule laufen. Der Kationenaustauscher ist mit dem gewünschten Kation beladen, z. B. mit Ammoniumionen, die sich von einem primären, sekundären oder tertiären Amin ableiten. Das gewünschte Salz erhält man durch Eindampfen des Eluats.

Ammoniumsalze der Dihydroxysäuren die sich von einem primären, sekundären oder tertiären Amin ableiten, kann man auch herstellen, indem man die freien Dihydroxysäuren in einer alkoholischen Lösung mit einer äquimolaren Menge des entsprechenden Amins versetzt und das Lösungsmittel eindampft.

Die freien Dihydroxysäuren II der δ-Lactone I lassen sich nach üblichen Methoden z.B. mit einem Diazoalkan verestern. So kann man z.B. Verbindungen der Formel I bei Temperaturen zwischen -40°C und +20°C mit einem Diazoalkan verestern, wobei die üblichen Lösungsmittel wie z.B. Diethylether, Tetrahydrofuran, Chloroform oder niedermolekulare Alkohole wie Methanol verwendet werden können. Die resultierenden Ester können in einfacher Weise durch Eindampfen des Lösungsmittels isoliert und ggf. chromatographisch gereinigt werden. Eine weitere Veresterungsmethode besteht darin, daß man Salze der Dihydroxysäuren II in Gegenwart einer Base wie z.B. eines Metallalkoholates oder Metallcarbonates in einem geeigneten Lösungsmittel mit einem Alkylierungsmittel umsetzt. Als Metallalkoholat kommen z.B. Natriumethylat oder Kaliumtertiärbutylat in Betracht. Als geeignetes Lösungsmittel kommen Alkohole wie z.B. Methanol oder tert. Butanol, Ether wie Tetrahydrofuran oder 1,2-Dimethoxyethan und insbesondere dipolare aprotische

Lösungsmittel wie Dimethylformamid, Dimethylsulfoxid, Acetonitril, oder N-Methylpyrrolidon in Betracht. Zur Darstellung von Estern der Dihydroxysäuren eignet sich auch die Methode der Umesterung mit Überschuß an Alkoholen wie z.B. Methanol, Ethanol, Isopropanol.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation, Säulen-, Dünnschicht- oder Hochdruckflüssigkeitschromatographie.

Falls der Aldehyd der Formel IV nicht als reines Enantiomeres vorliegt, können auch Mischungen der enantiomeren Endprodukte entstehen, die nach allgemein üblichen Verfahren aufgetrennt werden können.

Bei der Synthese von Verbindungen der allgemeinen Formeln I und II, worin $R^1$, $R^2$ und $R^3$ unabhängig voneinander Hydroxygruppen enthalten, ist es zweckmäßig, Ausgangsverbindungen der allgemeinen Formeln VIII-XII zu verwenden, in denen die Hydroxygruppen in geeigneter Weise geschützt sind, z.B. als Alkyl oder Silylether. Im erfindungsgemäßen Verfahren erhält man dann Verbindungen der allgemeinen Formeln I oder II, in denen $R^1$, $R^2$ oder $R^2$ die entsprechend geschützten Hydroxyfunktionen enthalten. Durch Abspaltung der Schutzgruppen nach literaturbekannten Verfahren lassen sich diese in Verbindungen der allgemeinen Formel I mit hydroxysubstituierten Resten $R^1$, $R^2$ oder $R^3$ überführen. Geeignete Schutzgruppen, sowie Methoden zu ihrer Einführung und Entfernung sind literaturbekannt (vgl. z.B. T.W. Greene, Protective Groups in Organic Synthesis, Wiley and Sons, New York, 1981).

In seltenen Fällen, in denen Verbindungen der allgemeinen Formeln I und II mit säureempfindlichen Resten $R^1$, $R^2$ oder $R^3$ hergestellt werden sollen, kann dies auch nach dem in der Patentanmeldung P 37 22 807.2 beschriebenen Verfahren geschehen.

Außer den in den Beispielen beschriebenen Verbindungen lassen sich nach den erfindungsgemäßen Verfahren die folgenden Verbindungen herstellen:

E-6S-(2-(2-Cyclohexyl-4-(4-fluorphenyl)-6-phenylpyridin-3-yl)-ethenyl)-4R-hydroxy-3,4,5,6-tetrahydro-2H-pyran-2-on
E-6S-(2-(4-Cyclohexyl-2-(4-fluorphenyl)-6-phenylpyridin-3-yl)ethenyl)-4R-hydroxy-3,4,5,6-tetrahydro-2H-pyran-2-on
E-6S-(2-(4-(3,5-Dimethylphenyl)-2-(1-methylethyl-6-phenylpyridin-3-yl)-ethenyl)-4R-hydroxy-3,4,5,6-tetrahydro-2H-pyran-2-on
E-6S-(2-(2-(3,5-Dimethylphenyl)-2-(1-methylethyl-6-phenylpyridin-3-yl)-ethenyl)-4R-hydroxy-3,4,5,6-tetrahydro-2H-pyran-2-on
E-6S-(2-(4,6-Diphenyl-2-(1-methylethyl)pyridin-3-yl)ethenyl)-4R-hydroxy-3,4,5,6-tetrahydro-2H-pyran-2-on
E-6S-(2-(2,6-Diphenyl-2-(1-methylethyl)pyridin-3-yl)ethenyl)-4R-hydroxy-3,4,5,6-tetrahydro-2H-pyran-2-on
E-6S-(2-(2-(1-Methylethyl)-6-phenyl-4-(4-trifluormethylphenyl)-pyridin-3-yl)-ethenyl)-4R-hydroxy-3,4,5,6-tetrahydro-2H-pyran-2-on
E-6S-(2-(4-(1-Methylethyl)-6-phenyl-4-(4-trifluormethylphenyl)-pyridin-3-yl)ethenyl)-4R-hydroxy-3,4,5,6-tetrahydro-2H-pyran-2-on
E-6S-(2-(4-(4-Fluor-3-methylphenyl)-2-(1-methylethyl)-6-phenylpyridin-3-yl)ethenyl)-4R-hydroxy-3,4,5,6-tetrahydro-2H-pyran-2-on
E-6S-(2-(2-(4-Fluor-3-methylphenyl)-2-(1-methylethyl)-6-phenylpyridin-3-yl)-ethenyl)-4R-hydroxy-3,4,5,6-tetrahydro-2H-pyran-2-on
E-6S-(2-(6-(4-Fluorphenyl)-2-(1-methylethyl)-4-phenylpyridin-3-yl)ethenyl)-4R-hydroxy-3,4,5,6-tetrahydro-2H-pyran-2-on
E-6S-(2-(6-(4-Fluorphenyl)-4-(1-methylethyl)-2-phenylpyridin-3-yl)ethenyl)-4R-hydroxy-3,4,5,6-tetrahydro-2H-pyran-2-on
E-6S-(2-(6-(3,5-Dimethylphenyl)-4-(4-fluorphenyl)2-(1-methylethyl)pyridin-3-yl)ethenyl)-4R-hydroxy-3,4,5,6-tetrahydro-2H-pyran-2-on
E-6S-(2-(6-(3,5-Dimethylphenyl)-2-(4-fluorphenyl)-4-(1-methylethyl)pyridin-3-yl)-ethenyl)-4R-hydroxy-3,4,5,6-tetrahydro-2H-pyran-2-on
E-6S-(2-(4,6-Bis-(1-methylethyl)-2-(4-fluorphenyl)pyridin-3-yl)ethenyl)-4R-hydroxy-3,4,5,6-tetrahydro-2H-pyran-2-on
E-6S-(2-(2,6-Bis-(1-methylethyl)-4-(4-fluorphenyl)pyridin-3-yl)ethenyl)-4R-hydroxy-3,4,5,6-tetrahydro-2H-pyran-2-on
E-6S-(2-(4-(4-Fluorphenyl)-2-(1-methylethyl)-6-(4-trifluormethylphenyl)pyridin-3-yl)ethenyl)-4R-hydroxy-3,4,5,6-tetrahydro-2H-pyran-2-on
E-6S-(2-(2-(4-Fluorphenyl)-4-(1-methylethyl)-6-(4-trifluormethylphenyl)pyridin-3-yl)ethenyl)-4R-hydroxy-3,4,5,6-tetrahydro-2H-pyran-2-on
E-6S-(2-(6-(4-Fluorphenyl)-4-(4-methoxyphenyl)-2-(1-methylethyl)pyridin-3-yl)ethenyl)-4R-hydroxy-3,4,5,6-tetrahydro-2H-pyran-2-on
E-6S-(2-(6-(4-Fluorphenyl)-2-(4-methoxyphenyl)-2-(1-methylethyl)-pyridin-3-yl)ethenyl)-4R-hydroxy-3,4,5,6-tetrahydro-2H-pyran-2-on
E-6S-(2-(2,6-Bis(1,1-dimethylethyl)-4-(4-fluorphenyl)pyridin-3-yl)ethenyl)-4R-hydroxy-3,4,5,6-tetrahydro-2H-pyran-2-on
E-6S-(2-(4,6-Bis(1,1-dimethylethyl)-2-(4-fluorphenyl)pyridin-3-yl)ethenyl)-4R-hydroxy-3,4,5,6-tetrahydro-2H-pyran-2-on

E-6S-(2-(4-(1,1-Dimethylethyl)-2-(4-fluorphenyl)-6-phenylpyridin-3-yl)ethenyl)-4R-hydroxy-3,4, 5, 6-tetrahydro-2H-pyran-2-on
E-6S-(2-(2-(4-Methoxyphenyl)-4-(1-methylethyl)-6-phenylpyridin-3-yl)ethenyl)-4R-hydroxy-3,4,5,6-tetrahydro-2H-pyran-2-on
E-6S-(2-(6-(2,5-Dimethylphenyl)-2-(4-fluorphenyl)-4-(1-methylethyl)pyridin-3-yl)ethenyl)-4R-hydroxy-3,4,5,6-tetrahydro-2H-pyran-2-on
E-6S-(2-(2,4-Bis-(4-fluorphenyl)-4-(1-methylethyl)pyridin-3-yl)-ethenyl)-4R-hydroxy 3,4,5,6-tetrahydro-2H-pyran-2-on
E-6S-(2-(6-Cyclohexyl-4-(4-fluorphenyl)-2-(1-methylethyl)pyridin-3-yl)-ethenyl)-4R-hydroxy-3,4,5,6-tetrahydro-2H-pyran-2-on
E-6S-(2-(6-Cyclohexyl-2-(4-fluorphenyl)-4-(1-methylethyl)pyridin-3-yl)ethenyl)-4R-hydroxy-3,4,5,6-tetrahydro-2H-pyran-2-on
E-6S-(2-(4-(4-Fluorphenyl)-2-(1R-methylpropyl)-6-phenylpyridin-3-yl)ethenyl)-4R-hydroxy-3,4,5,6-tetrahydro-2H-pyran-2-on
E-6S-(2-(4-(4-Fluorphenyl)-2-(1S-methylpropyl)-6-phenylpyridin-3-yl)ethenyl)-4R-hydroxy-3,4,5,6-tetrahydro-2H-pyran-2-on
E-6S-(2-(2-(4-Fluorphenyl)-4-(1R-methylpropyl)-6-phenylpyridin-3-yl)ethenyl)-4R-hydroxy-3,4,5,6-tetrahydro-2H-pyran-2-on
E-6S-(2-(2-(4-Fluorphenyl)-4-(1S-methylpropyl)-6-phenylpyridin-3-yl)ethenyl)-4R-hydroxy-3,4,5,6-tetrahydro-2H-pyran-2-on
E-6S-(2-(4-(4-Fluorphenyl)-2-(1-methylethyl)-6-phenylpyridin-3-yl)-ethyl)-4R-hydroxy-3,4,5,6-tetrahydro-2H-pyran-2-on
E-6S-(2-(2-(4-Fluorphenyl)-4-(1-methylethyl)-6-phenylpyridin-3-yl)-ethyl)-4R-hydroxy-3,4,5,6-tetrahydro-2H-pyran-2-on
E-6S-(2-(2-Cyclohexyl-4-(4-fluorphenyl)-6-phenylpyridin-3-yl)ethyl)-4R-hydroxy-3,4,5,6-tetrahydro-2H-pyran-2-on
E-6S-(2-(4-(4-Methoxyphenyl)-2-(1-methylethyl)-6-phenyl)ethyl)-4R-hydroxy-3,4,5,6-tetrahydro-2H-pyran-2-on
E-6S-(2-(6-(2,5-Dimethylphenyl)-4-(4-fluorphenyl)-2-(1-methylethyl)pyridin-3-yl)ethyl)-4R-hydroxy-3,4,5,6-tetrahydro-2H-pyran-2-on
E-6S-(2-(6-(3,5-Dimethylphenyl)-4-(4-fluorphenyl)-2-(1-methylethyl)pyridin-3-yl)ethyl)-4R-hydroxy-3,4,5,6-tetrahydro-2H-pyran-2-on
E-6S-(2-(5-(2-Phenyl-4-(4-flurophenyl)-6-isopropyl)-pyrimidinyl)ethenyl)-4R-hydroxy-3,4,5,6-tetrahydro-2H-pyran-2-on
E-6S-(2-(5-(2-(2-Methylphenyl)-4-(4-chlorphenyl)-6-isopropyl)-pyrimidinyl)ethenyl)-4R-hydroxy-3,4,5,6-tetrahydro-2H-pyran-2-on
E-6S-(2-(5-(2-(2,6-Dimethylphenyl)-4-(4-fluorphenyl)-6-isopropyl)-pyrimidinyl)ethenyl)-4R-hydroxy-3,4,5,6-tetrahydro-2H-pyran-2-on
E-6S-(2-(5-(2-(2,6-Dichlorphenyl)-4-(4-fluorphenyl)-6-isopropyl)-pyrimidinyl)ethenyl)-4R-hydroxy-3,4,5,6-tetrahydro-2H-pyran-2-on
E-6S-(2-(5-(2-Phenyl-4-(4-chlorphenyl)-6-t-butyl)-pyrimidinyl)ethenyl)-4R-hydroxy-3,4,5,6-tetrahydro-2H-pyran-2-on
E-6S-(2-(5-(2-Phenyl-4-(4-fluorphenyl)-6-t-butyl)-pyrimidinyl)ethyl)-4R-hydroxy-3,4,5,6-tetrahydro-2H-pyran-2-on
E-6S-(2-(5-(2-Phenyl-4-(4-fluor-3-methyl-phenyl)-6-isopropyl-pyrimidinyl)-ethenyl)-4R-hydroxy-3,4,5,6-tetrahydro-2H-pyran-2-on
E-6S-(2-(5-(2,6-Diisopropyl-4-(4-fluorphenyl)-pyrimidinyl)ethenyl)-4R-hydroxy-3,4,5,6-tetrahydro-2H-pyran-2-on
E-6S-(2-(5-(2-Phenyl-4-(4-fluor-3-methyl-phenyl)-6-isopropyl-pyrimidinyl)ethyl)-4R-hydroxy-3,4,5,6-tetrahydro-2H-pyran-2-on
E-6S-(2-(5-(2,6-Diisopropyl-4-(4-chlorphenyl)-pyrimidinyl)ethenyl)-4R-hydroxy-3,4,5,6-tetrahydro-2H-pyran-2-on
E-6S-(2-(5-(2,6-Diisopropyl-4-(4-methoxyphenyl)-pyrimidinyl)ethenyl)-4R-hydroxy-3,4,5,6-tetrahydro-2H-pyran-2-on
E-6S-(2-(5-(2,6-Diisopropyl-4-cyclohexyl)pyrimidinyl)ethenyl)-4R-hydroxy-3,4,5,6-tetrahydro-2H-pyran-2-on
E-6S-(2-(5-(2-Phenyl-4-cyclohexyl-6-isopropyl)pyrimidinyl)ethenyl)-4R-hydroxy-3,4,5,6-tetrahydro-2H-pyran-2-on
E-6S-(2-(5-(2,6-Ditert.-butyl-4-(4-chlorphenyl)pyrimidinyl)ethenyl)-4R-hydroxy-3,4,5,6-tetrahydro-2H-pyran-2-on
E-6S-(2-(5-(2,6-Ditert.butyl-4-(4-fluorphenyl)pyrimidinyl)ethenyl)-4R-hydroxy-3,4,5,6-tetrahydro-2H-pyran-2-on
E-6S-(2-(5-(2-(2,6-Dichlorphenyl)-4-(4-fluorphenyl)-6-isopropyl)pyrimidinyl)-ethenyl)-4R-hydroxy-3,4,5,6-tetrahydro-2H-pyran-2-on
E-6S-(2-(5-(2-(2-Chlor-4-methyl-phenyl)-4-(4-chlorphenyl)-6-isopropyl)-pyrimidinyl)ethenyl)-4R-hydroxy-3,4,5,6-tetrahydro-2H-pyran-2-on
E-6S-(2-(5-(2-(2,4-Dichlorphenyl)-4-(4-fluorphenyl)-6-methyl)pyrimidinyl)ethenyl)-4R-hydroxy-3,4,5,6-tetrahydro-2H-pyran-2-on
E-6S-(2-(5-(2-(2,4-Dimethyl-phenyl)-4-(4-methoxyphenyl)-6-isopropyl)-pyrimidinyl)ethyl)-4R-hydroxy-3,4,5,6-tetrahydro-2H-pyran-2-on
E-6S-(2-(5-(2-(2-Chlor-4-methyl-phenyl)-4-(4-fluor-3-phenyl)6-isoproyl)-pyrimidinyl)ethyl)-4R-hydroxy-3,4,5,6-tetrahydro-2H-pyran-2-on

E-6S-(2-(5-(2-Phenyl-4-(4-fluorphenyl)-6-isopropyl)-pyrimidinyl)-ethyl)-4R-hydroxy-3,4,5,6-tetrahydro-2H-pyran-2-on
E-6S-(2-(5-(2-Phenyl-4-(4-fluro-3-methyl-phenyl)-6-tert.butyl)pyrimidinyl)-ethyl)-4R-hydroxy-3,4,5,6-tetrahydro-2H-pyran-2-on

Biologische Testsysteme:

1. HMG-COA-Reduktase-Aktivität in Enzympräparationen

Die HMG-COA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden.

Als Substrat diente (S,R) $^{14}$C-HMG-COA, die Konzentration von NADPH wurde während der Inkubation durch ein regenerierendes System aufrechterhalten. Die Abtrennung von $^{14}$C-Mevalonat vom Substrat und anderen Produkten (z.B. $^{14}$C-HMG) erfolgte über Säulenelution, wobei das Elutionsprofil jeder Einzelprobe ermittelt wurde. Auf die ständige Mitführung von $^{3}$H-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz ( = 100 %) und solche mit Präparatezusätzen, Endkonzentration $10^{-5}$ bis $10^{-9}$ M, zusammen behandelt. Jeder Einzelwert wurde als Mittelwert aus 3 Parallelproben gebildet. Die Signifkanz der Mittelwertsunterschiede zwischen präparatefreien und präparatehaltigen Proben wurde nach dem t-Test beurteilt.

Nach der oben beschriebenen Methode wurden von den erfindungsgemäßen Verbindungen z.B. folgende Hemmwerte auf die HMG-COA-Reduktase ermittelt [$IC_{50}$/mol/Liter bedeutet die molare Konzentration der Verbindung pro Liter, die für eine 50 %ige Hemmung erforderlich ist]:

| Verb. gem. Bsp. | Z | $R^1$ | $R^2$ | $R^3$ | A-B | $IC_{50}$/mol/Liter |
|---|---|---|---|---|---|---|
| 13a | CH | $iC_3H_7$ | $4\text{-}FC_6H_4$ | $C_6H_5$ | (E)-CH = CH- | $2.9 \cdot 10^{-9}$ |
| 13b | CH | $4\text{-}FC_6H_4$ | $iC_3H_7$ | $C_6H_5$ | (E)-CH = CH- | $4.0 \cdot 10^{-9}$ |
| 13c | CH | $tC_4H_9$ | $4\text{-}FC_6H_4$ | $C_6H_5$ | (E)-CH = CH- | $1.8 \cdot 10^{-8}$ |
| 13d | CH | $iC_3H_7$ | $4\text{-}FC_6H_4$ | 2.5-$(CH_3)_2C_6H_4$ | (E)-CH = CH- | $5.0 \cdot 10^{-9}$ |
| 13e | CH | $iC_3H_7$ | $4\text{-}FC_6H_4$ | $4\text{-}FC_6H_4$ | (E)-CH = CH- | $2.3 \cdot 10^{-9}$ |
| 13f | N | $iC_3H_7$ | $4\text{-}FC_6H_4$ | $C_6H_5$ | (E)-CH = CH- | $3.0 \cdot 10^{-9}$ |
| 13g | CH | $iC_3H_7$ | $4\text{-}FC_6H_4$ | $iC_3H_7$ | (E)-CH = CH- | $2.5 \cdot 10^{-9}$ |
| 13h | CH | $iC_3H_7$ | $4\text{-}FC_6H_4$ | $tC_4H_9$ | (E)-CH = CH- | $1.2 \cdot 10^{-9}$ |
| 13i | CH | $iC_3H_7$ | $4\text{-}FC_6H_4$ | $cC_6H_{11}$ | (E)-CH = CH- | $3.7 \cdot 10^{-9}$ |
| 13j | N | $iC_3H_7$ | $4\text{-}FC_6H_4$ | $iC_3H_7$ | (E)-CH = CH- | $2.5 \cdot 10^{-9}$ |
| 13k | N | $iC_3H_7$ | $4\text{-}FC_6H_4$ | $4\text{-}FC_6H_4$ | (E)-CH = CH- | $0.9 \cdot 10^{-9}$ |
| 13l | N | $iC_3H_7$ | $4\text{-}FC_6H_4$ | $C_6H_5$ | $-CH_2\text{-}CH_2-$ | $3.3 \cdot 10^{-9}$ |
| 13m | CH | $iC_3H_7$ | $4\text{-}FC_6H_4$ | $4\text{-}HOC_6H_4$ | (E)-CH = CH- | $1.5 \cdot 10^{-9}$ |
| 13n | CH | $cC_3H_5$ | $4\text{-}FC_6H_4$ | $C_6H_5$ | (E)-CH = CH- | $1.0 \cdot 10^{-9}$ |

2. Supprimierung bzw. Inhibierung der HMG-CoA-Reduktase in Zellkulturen von HEP-G2-Zellen

Monolayers von HEP-G2-Zellen in lipoproteinfreiem Nährmedium wurden mit entsprechenden Konzentrationen der Prüfsubstanzen eine bestimmte Zeit (z.B. 1 Stunde) vorinkubiert, nach Zugabe des markierten Präkursors, z.B. $^{14}$C-Natriumacetat wurde die Inkubation fortgesetzt (z.B. 3 Stunden). Nach Zugabe eines internen Standards ($^{3}$H-Cholesterin) wurde ein Teil der Zellen alkalisch verseift. Die Lipide der verseiften Zellen wurden mit Chloroform/Methanol extrahiert. Dieses Lipidgemisch wurde nach Zusatz von Träger-Cholesterin präparativ dünnschichtchromatographisch getrennt, die Cholesterinbande nach dem Sichbarmachen mit Jod-Dampf isoliert und die aus dem $^{14}$C-Präkursor gebildete Menge $^{14}$C-Cholesterin szintigraphisch bestimmt. In einem aliquoten Teil der Zellen wurde Zellprotein bestimmt,

so daß die in der Zeiteinheit pro mg Zellprotein gebildete Menge $^{14}C$- Cholesterin berechnet werden kann. Durch Vergleich dieses Wertes mit der Menge an $^{14}C$-Cholesterin, die pro mg Zellprotein und Zeiteinheit in einer gleichbehandelten, jedoch prüfsubstanzfreien Kultur gebildet wurde, ergab sich die Hemmwirkung des jeweiligen Prüfpräparates auf die Cholesterin-Biosynthese von HEP-G2-Zellkulturen.

Prüfung von Substanzen auf
Hemmung der Cholesterin-Biosynthese in Zellkulturen

Konfluente Zellkultur (Monolayer) von HEP-G2-Zellen

1. liporoteinfreies Medium (DMEM) — 24 h
2. Inkubation mit Prüfpräparaten — 1 h
3. Inkubation mit $^{14}C$-Acetat — 3 h
4. Cytolyse
5. DC-Trennung des Reaktionsprodukts $^{14}C$-Cholesterin
6. Isolierung des $^{14}C$-Cholesterin
7. Szintillationsmessung

8. Ergebnis
in nmol $^{14}C$-Cholesterin/mg Zellprotein im Vergleich zur Lösungsmittelkontrolle

Nach der oben beschriebenen Methode wurden von den erfindungsgemäßen Verbindungen z.B. folgende Hemmwerte der Cholesterinbiosynthese (in HEP-G2-Zellen) ermittelt (der $IC_{50}$/mol ist diejenige Konzentration der Verbindung, die eine 50 %ige Hemmung der Cholesterinbiosynthese bewirkt) (Tab. 2):

| Verb. gem. Bsp. | Z | $R^1$ | $R^2$ | $R^3$ | A-B | $IC_{50}$/mol/Liter |
|---|---|---|---|---|---|---|
| 11a | CH | i-$C_3H_7$ | 4-F-$C_6H_4$ | $C_6H_5$ | (E)-CH = CH- | $5 \cdot 10^{-9}$ |
| 11g | N | i-$C_3H_7$ | 4-F-$C_6H_4$ | $C_6H_5$ | (E)-CH = CH- | $5 \cdot 10^{-9}$ |

Die Verbindungen der allgemeinen Formel I bzw. II zeichnen sich aus durch starke Hemmung der HMG-CoA-Reduktase, des geschwindigkeitsbestimmenden Enzyms der Cholesterinbiosynthese`

Das durch $IC_{50}$-Werte im Bereich von $10^{-7}$ - $10^{-9}$ mol pro Liter charakterisierte Ausmaß der Hemmung ist bei Verbindungen der allgemeinen Formel I bzw. II deutlich höher als bei literaturbelkannten, vollsynthetischen HMG-CoA-Reduktase- Inhibitoren wie z.B. den von G.E. Stokker et al., J. Med. Chem. 29, 170 (1986) beschriebenen.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J.R. Sabine in CRC Series in Enzyme Biology: 3-Hydroxy-3-methylglutaryl Coenzym A Reductase, CRC Press Inc. Boca Raten, Florida 1983 (ISBN 0-8493-6551-1)).

Hohe Cholesterinspiegel werden mit einer Reihe von Erkrakungen, wie z.B. koronarer Herzkrankheit oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbivsynthese. Hemmstoffe der HMG-CoA-Reduktase blockieren die Cholesterinbiosynthese auf einer frühen Stufe.

Die Verbindungen der allgemeinen Formel I bzw. II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formel I bzw. II als Hypolipidemika oder Anti-arteriosklerotika erfolgt in oralen Dosen von 3 bis 2500 mg, vorzugsweise jedoch im Dosisbereich von 10-500 mg. Diese Tagesdosen können nach Bedarf auch in zwei bis vier Einzeldosen aufgeteilt werden oder in Retardform verabreicht werden. Das Dosierungsschema kann vom Typ, Alter, Gewicht, Geschlecht und medizinischen Zustand des Patienten abhängen.

Ein zusätzlicher cholesterinsenkender Effekt läßt sich durch gleichzeitige Gabe der erfindungsgemäßen Verbindungen mit gallensäurebindenen Stoffen, wie z.B. Anionenaustauscherharzen, erzielen. Die Gallensäureausscheidung führt zu einer verstärkten Neusynthese und damit zu einem erhöhten Cholesterinabbau (vgl. M.S. Brown, P.T. Koranen und J.C. Goldstein, Science 212, 628 (1981); M.S. Brown, J.C. Goldstein, Spektrum der Wissenschaft 1985, 1,96).

Die erfindungsgemäßen Verbindungen der Formel I bzw. II können in Form der δ-Lactone, als freie Säuren oder in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyddiacetalgemischen, Ölen wie z.B. Sonnenblumenöl oder Lebertran, Ethern, wie z.B. Diethylenglykoldimethylether, oder auch Polyethern, wie z.B. Polyethylenglykol, oder auch in Gegenwart anderer pharmakologisch unbedenklicher Polymerträger wie z.B. Polyvinylpyrrolidon oder in festen Zubereitungen zur Anwendung gelangen.

Für die Verbindungen der Formel I bzw. II sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

Die Verbindungen der Formel III, V, VI und VII sind neu und stellen wertvolle Zwischenprodukte für die Herstellung von Verbindungen der Formel I dar. Die Erfindung betrifft daher auch diese Verbindungen sowie Verfahren zu ihrer Herstellung.

Vorbemerkung: NMR-Spektren wurden, sofern nichts anderes angegeben, in $CDCl_3$ mit internem Standard TMS gemessen.

Zur Klassifizierung von NMR-Signalen gelten folgende Abkürzungen:

s = Singulett, brs = breites Singulett, d = Dublett, t = Triplett, q = Quartett, h = Heptett, mc = zentriertes Multiplett, m = Multiplett.

Schmelzpunkte sind unkorrigiert.

Es gelten folgende Substituentenabkürzungen:

i = iso, t = tertiär, c = cyclo.

Beispiel 1

Allgemeine Vorschrift zur Herstellung der Verbindungen der allgemeinen Formel X

Versuchsvorschrift 1 ($R^1 = CH_3$, $R^2 = 4\text{-}FC_6H_4$, $R^3 = CH_3$, $R^{10} = CH_3$) 2-(1-Oxoethyl)-3-(4-fluorphenyl)-5-oxohexansäuremethylester

58.1 g (0.50 mol) Acetessigsäuremethylester wurden mit einer Lösung von 1.2 g Kaliumhydroxid in 12 ml Ethanol versetzt. Anschließend wurde eine Lösung von 41.0 g (0.25 mol) 4-(4-Fluorphenyl)but-3-en-2-on in 600 ml Ether so langsam zugetropft, daß die Temperatur des Reaktionsgemisches 30°C nicht überstieg. Nach dreistündigem Stehen bei Raumtemperatur wurde der Ansatz mit 1 l Ether verdünnt und durch Zusatz von Essigsäure auf einen pH-Wert von 5 gebracht. Er wurde nacheinander mit Wasser und gesättigter $NaHCO_3$-Lösung ausgeschüttelt, über $MgSO_4$ getrocknet und eingedampft.

| | |
|---|---|
| Ausbeute: | 50.6 g (72 %) |
| Fp.: | Öl |
| $1^H$-NMR: | δ/ppm = 0.8-1.0 (m, 6H), 1.9 (S, 3H), 2.2-2.9 (m, 2H), 3.1-4.1 (m, 7H), 7.0-7.8 (m, 4H). |

Beispiel 1a-1i

Die Verbindungen Xa-Xi wurden in analoger Weise wie in Versuchsvorschrift 1 beschrieben, dargestellt (vgl. Tabelle 3)

Tabelle 3

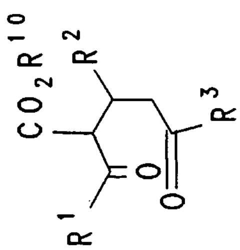

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| a | X a | $iC_3H_7$ | $4\text{-}FC_6H_4$ | $C_6H_5$ | $C_2H_5$ | 69 | $0.23^b$ Öl | 0.6-1.3 (m,9H), 2.2-2.9 (m,1H), 3.2-3.6 (m,2H), 3.3-4.4 (m,4H), 6.8-9.0 (m,9H) 385 ($M^+$ +H). |

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| b | X b | $4FC_6H_4$ | $iC_3H_7$ | $C_6H_5$ | $CH_3$ | 71 | $0.38^c$ 60 | 0.7-1.1 (m,6H), 1.6-2.1 (m,1H), 2.9-3.4 (m,2H), 3.6 (s,3H), 4.0-4.8 (m,2H), 7.0-7.7 (m,5H), 7.9-8.2 (m,4H). 370 $M^+$) |
| c | X c | $iC_3H_7$ | $4-CH_3OC_6H_4$ | $C_6H_5$ | $C_2H_5$ | 75 | $0.32^c$ 81 | 0.6-2.1 (m,9H), 2.1-2.8 (m,1H), 3.2-3.5 (m,2H), 3.8 (s,3H), 3.94.4 (m,4H), 7.0 (m,4H), 7.3-7.6 (m, 3H), 7.8-8.0 (m,2H) 396 ($M^+$) |
| d | X d | $iC_3H_7$ | $4-F-C_6H_4$ | $2,5(CH_3)_2-C_6H_3$ | $C_2H_5$ | 87 | $0.44^c$ Öl | 0.6-1.4 (m,9H), 2.1 (s,3H), 2.4 (s,3H), 2.5-2.9 (m,1H), 3.1-3.4 (m,2H), 3.7-4.4 (m,4H), 6.8-7.4 (m,7H) 413 ($M^+$ +H) |

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| e | X e | $iC_3H_7$ | $4\text{-}FC_6H_4$ | $4\text{-}FC_6H_4$ | $C_2H_5$ | 70 | 0.63[a] 122 | 0.7-1.4 (m,9H), 2.2-2.9 (m,1H), 3.2-3.6 (m,2H), 3.9-4.4 (m,4H), 6.8-7.4 (m,6H), 7.9-8.1 (m,2H) |
| f | X f | $iC_3H_7$ | $4\text{-}FC_6H_4$ | $iC_3H_7$ | $C_2H_5$ | 61 | 0.25[e] 49-51 | 0.7-1.4 (m,15H), 2.4 (mc,2H), 2.9 (mc,2H), 3.8-4.4 (m,4H), 7.1 (mc, 4H) 351 ($M^+$ +H |
| g | X g | $iC_3H_7$ | $4\text{-}FC_6H_4$ | $tC_4H_9$ | $C_2H_5$ | 55 | 0.25[e] 72-75 | 0.6-1.3 (m 18H), 2.3-3.1 (m,3H), 3.7-4.4 (m,4H), 6.9-7.2 (m,4H) 307 ($M^+$ - $C_4H_9$) |
| h | X h | $iC_3H_7$ | $4\text{-}FC_6H_4$ | $cC_6H_{11}$ | $C_2H_5$ | 47 | 0.58[d] 99-101 | 0.8-1.9 (m,19H), 2.0-2.6 (m2H), 2.7-3.0 (m,2H), 3.8-4.4 (m,4H), 6.8-7.4 (m,4H) 391 ($M^+$ +H) |

| Bsp. | Verb. | $R^1$ | $R^2$ | $R^3$ | $R^{10}$ | Ausb. % | $R_f$-Wert<br>Fp°C | $^1$H-NMR: δ/ppm =<br>MS : m/e = |
|---|---|---|---|---|---|---|---|---|
| i | X i | $cC_6H_{11}$ | $4\text{-}FC_6H_4$ | $C_6H_5$ | $C_2H_5$ | 65 | 0.35(A);<br>0.3(B)[d]<br><br>137-139(A)<br>108-110(B) | A: 0.8-1.3 (m,9H), 1.5-1.8 (m, 4H), 2.2 (mc,1H), 3.3-3.5 (m,2H), 4.1-4.3 (m,4H), 6.9 (mc,2H), 7.2-7.6 (m,6H), 7.9 (mc,2H)<br>B: 1.0 (t,J=7Hz,3H), 1.2-1.9 (m, 10H), 2.5 (mc,1H), 3.2-3.4 (m, 2H), 3.9 (q,J=7Hz, 2H), 4.2 (mc, 2H), 6.9 (mc, 2H), 7.1-7.6 (m,3H), 7.9 (mc,2H)<br>425 ($M^+$+H) |

[a]Cyclohexan/Ethylacetat 2:1 [b]Cyclohexan/Ethylacetat 8:1 [c]Cyclohexan/Ethylacetat 3:1
[d]Cyclohexan/Ethylacetat 4:1 [e]Cyclohexan/Ethylacetat 10:1

Beispiel 2

Allgemeine Vorschrift zur Darstellung der Verbindungen der allgemeinen Formel XV (Z = CH) (vgl. F. Rehberg, F. Kröhnke, Liebigs Ann. Chem. 717, 91 (1968))

Versuchsvorschrift 2 ($R^1 = CH_3$, $R^2 = 4\text{-}FC_6H_4$, $R^3 = CH_3$, $R^{10} = CH_3$) 2,6-Dimethyl-4-(4-fluorphenyl)pyridin-3-carbonsäuremethylester

Eine Suspension von 28.0 g (100 mmol) 3-(4-Fluorphenyl)-2-(1-oxoethyl)-5-oxohexansäuremethylester , 120 g Ammoniumacetat und 120 g Eisen(III)-chloridhexahydrat in 1000 ml Eisessig wurde unter Rühren so lange refluxiert, bis dünnschichtchromatographisch kein Edukt mehr nachgewiesen werden konnte (4 h). Nach Abkühlen wurde filtriert, der feste Rückstand mit Ethanol und Toluol gewaschen. Nach Eindampfen des Filtrats wurde der Rückstand in Wasser suspendiert, durch Zusatz von festem Natriumhydrogencarbonat auf pH 8-9 gebracht und mehrfach mit Ether extrahiert. Die vereinigten Extrakte wurden mit gesättigter Kochsalzlösung gewaschen, über $MgSO_4$ getrocknet und eingedampft. Eine säulenchromatographische Reinigung des Rohprodukts (Kieselgel, Cyclohexan/Ethylacetat 2:1) lieferte 23.6 g (91 %) XVa in Form weißer Kristalle.

| | |
|---|---|
| Fp.: | 89-90°C |
| $^1$H-NMR ($CDCl_3$; 60 MHz): | δ/ppm = 2.60 (s, 6H), 3.66 (s, 3H), 6.95-7.50 (m, 5H). |
| MS: | m/e = 259 ($M^+$) |

Beispiel 2a - 2j

Die Verbindungen XVa - XVj wurden in analoger Weise, wie in Versuchsvorschrift 2 beschrieben, dargestellt (vgl. Tabelle 4)

Tabelle 4

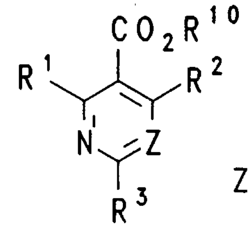

Z = CH

| Bsp. | Verb. | $R^1$ | $R^2$ | $R^3$ | $R^{10}$ | Ausb. % | $R_f$-Wert Fp°C | $^1$H-NMR: δ/ppm = MS : m/e = |
|---|---|---|---|---|---|---|---|---|
| a | XV a | $iC_3H_7$ | $4\text{-}FC_6H_4$ | $C_6H_5$ | $C_2H_5$ | 99 | 0.75[b] Öl | 1.1 (t,J=7Hz,3H), 1.3 (d,J=7Hz, 6H), 3.3 (h,J=7Hz, 1H), 4.2 (q, J= Hz,2H), 7.0-7.6 (m,8H), 8.1-8.2 (m, 2H). 363 ($M^+$) |
| b | XV b | $4\text{-}FC_6H_4$ | $iC_3H_7$ | $C_6H_5$ | $CH_3$ | 73 | 0.50[b] Öl | 1.3 (d,J=7Hz,6H), 3.2 (h,J= 7Hz, 1H), 3.7 (s,3H), 7.0-8.2 (m,10H). 349 ($M^+$) |

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| c | XV c | $tC_4H_9$ | $4\text{-}FC_6H_4$ | $C_6H_5$ | $C_2H_5$ | 85 | $0.66^c$ Öl | 1.0 (t,J=7Hz,3H), 1.4 (s,9H), 4.0 (q,J=7Hz,2H), 7.0-7.7 (m,8H), 8.1-8.3 (m,2H) 378 ($M^+$+H) |
| d | XV d | $iC_3H_7$ | $4\text{-}CH_3OC_6H_4$ | $C_6H_5$ | $C_2H_5$ | 88 | $0.54^c$ 72-74 | 1.1 (t,J=7Hz,3H), 1.4 (d,J=7Hz, 6H), 3.2 (h,J=7Hz,1H), 3.9 (s,3H), 4.2 (q,J=7Hz,2H), 6.9-7.6 (m,8H), 8.0-8.2 (m,2H), 376 ($M^+$+H) |
| e | XV e | $iC_3H_7$ | $4\text{-}FC_6H_4$ | $2,5\text{-}(CH_3)_2\text{-}C_6H_3$ | $C_2H_5$ | 91 | $0.63^c$ Öl | 1.1 (t,J=7Hz,3H), 1.4 (d,J=7Hz, 6H), 2.4 (s,3H), 2.5 (s,3H), 3.2 (h,J=7Hz,1H), 4.2 (q, J= HZ,2H), 7.0-7.6 (m,8H) 392 ($M^+$+H) |

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| f | XV f | $iC_3H_7$ | $4\text{-}FC_6H_4$ | $4\text{-}FC_6H_4$ | $C_2H_5$ | 78 | 0.58[c] 112-114 | 1.1 (t,J=7Hz,3H), 1.4 (d,J=7Hz, 6H), 3.2 (h,J=7Hz,1H), 4.2 (q, J=7Hz,2H), 7.0-7.6 (m,7H), 8.0-8.3 (m,2H) 381 ($M^+$+H) |
| g | XV g | $iC_3H_7$ | $4\text{-}FC_6H_4$ | $iC_3H_7$ | $C_2H_5$ | 89 | 0.73[d] | 1.1 (t,J=7Hz,3H), 1.3 (d,J=7Hz, 12H), 3.1 (mc,2H), 4.1 (q,J=7Hz, 2H), 7.0 (s,1H), 7.2 (mc,4H) |
| h | XV h | $iC_3H_7$ | $4\text{-}FC_6H_4$ | $tC_4H_9$ | $C_2H_5$ | 81 | 0.83[c] Öl | 0.9-1.4 (m,18H), 3.1 (h,J=7Hz,1H), 4.1 (q,J=7Hz,2H), 7.0-7.5 (m,5H) 344 ($M^+$+H) |
| i | XV i | $iC_3H_7$ | $4\text{-}FC_6H_4$ | $cC_6H_{11}$ | $C_2H_5$ | 96 | 0.76[c] Öl | 1.0 (t,J=7Hz,3H), 1.2-2.1 (m,16H), 2.5-3.4 (m,2H), 4.1 (q,J=7Hz,2H), 7.0 (s,1H), 7.2 (mc,2H) 369 ($M^+$) |

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| j | XV j | $cC_6H_{11}$ | $4\text{-}FC_6H_4$ | $C_6H_5$ | $C_2H_5$ | 84 | 0.55[d] Öl | 1.1 (t,J = 7Hz,3H), 1.1-2.0 (m,10H), 2.4-3.0 (m,1H), 4.2 (q,J = 7Hz,2H), 7.0-7.6 (m,8H), 8.1 (m,2H) 404 ($M^+$ + H) |

[a]Cyclohexan/Ethylacetat 1:1 [b]Cyclohexan/Ethylacetat 3:1 [c]Cyclohexan/Ethylacetat 8:1
[d]Cyclohexan/Ethylacetat 4:1

Beispiel 3

Allgemeine Vorschrift zur Herstellung von Verbindungen der allgemeinen Formel XIV

Versuchsvorschrift 3 ($R^1 = iC_3H_7$, $R^2$ = 4-$FC_6H_4$, $R_3 = C_6H_5$, $R^{10} = C_2H_5$) 1,4-Dihydro-4-(4-fluorphenyl)-2-(1-methylethyl)-6-phenylpyrimidin-3-carbonsäureethylester

Weg A

98.6 g (0.62 mol) 4-Methyl-3-oxopentansäuremethylester (entspr. der allgem. Formel IX, Schema 1) wurden in 400 ml Toluol gelöst und mit 77.0 g 4-Fluorbenzaldehyd XIa, 6.8 g Piperazin sowie 7.3 g Capronsäure versetzt. Das Reaktionsgemisch wurde so lange am Wasserabscheider unter Rückfluß gekocht, bis sich kein Reaktionswasser mehr bildete (24h) und anschließend dreimal mit gesättigter Natriumhydrogencarbonatlösung, dreimal mit 5 %iger Essigsäure und einmal mit Wasser ausgeschüttelt. Nach Trocknung über Magnesiumsulfat wurde die organische Phase eingedampft. Zurück blieben 150 g ( 0.58 mol) des rohen XII, das in 1700 ml Toluol gelöst und nacheinander mit 102.2 g (0.85 mol) Benzamidin $\cdot HCl\cdot H_2O$ XIII und 90.7 g (0.94 mol) Kaliumacetat versetzt wurde. Dieses Gemisch wurde bis zum Ende der Reaktionswasserbildung am Wasserabscheider unter Rückfluß gekocht (24h), anschließend mit Natriumhydrogencarbonatlösung, 5 %iger Essigsäure und Wasser gewaschen, über Magnesiumsulfat getrocknet und eingedampft.

Das erhaltene Rohprodukt (78 g) wurde über Kieselgel (Laufmittel Cyclohexan/Ethylacetat 4:1) filtriert.
Ausbeute: 72.6 (63 %) XIV als gelbes Öl
Rf-Wert (Cyclohexan/Ethylacetat 2:1) 0.31

$^1$H-NMR: δ/ppm = 1.2 (t,J= 7Hz,3H), 1.3 (d, J= 7Hz,6H), 4.0-4.5 (m,3H), 5.8 (s,1H), 7.0-7.9 (m,10H).

Weg B

98.6 g (0.62 mol) 4-Methyl-3-oxopentansäureethylester (IXa), 77.0 g (0.62 mol) 4-Fluorbenzaldehyd XIa 102 g (0.85 mol) Benzamidin$\cdot H_2O\cdot HCl$ (XIIIa) und 90.7 (0.94 mol) Kaliumacetat wurden in 1500 ml Toluol gelöst und 24 h am Wasserabscheider refluxiert. Aufarbeitung und Reinigung wie unter Weg A beschrieben lieferten XIVo als gelbes Öl.

| | |
|---|---|
| Ausbeute: | 110 g (55 %) |
| Rf-Wert: | vgl. Weg A |
| $^1$H-NMR: | vgl. Weg A |

Beispiel 3a - 3b

Die Verbindungen XIVa und XIVb wurden in analoger Weise, wie in Versuchsvorschrift 3 beschrieben, hergestellt (vgl.Tabelle 5)

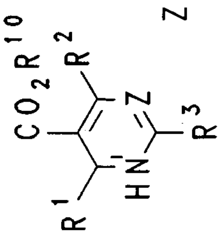

Z = N

Tabelle 5

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| a | XIVa | $iC_3H_7$ | $4\text{-}FC_6H_4$ | $iC_3H_5$ | 46 | 46 | $0.39^c$ 115-117° | 328 ($M^+$) |
| b | XIVb | $iC_3H_7$ | $4\text{-}FC_6H_4$ | $4\text{-}FC_6H_4$ | $C_2H_5$ | 80 | $0.39^c$ Öl | 384 ($M^+$) |

Beispiel 4

Allgemeine Vorschrift zur Herstellung von Verbindungen der allgemeinen Formel XV (Z=N)

Versuchvorschrift 4 ($R^1$ = $iC_3H_7$, $R^2$ = 4-$FC_6H_4$, $R^3$ = $C_6H_5$, $R^{10}$ = $C_2H_5$) 4-(4-Fluorphenyl)-2-(1-methylethyl)-6-phenylpyrimidin-3-carbonsäureethylester XV
24.2 g (66.0 mmol) 1,4-Dihydro-4-(4-fluorphenyl)-2-(1-methylethyl)6-phenylpyrimidin-3-carbonsäureethylester XIV (aus Beispiel 3a) wurden in 300 ml Toluol gelöst, mit 18.0 g (790 mmol) 2,3-Dichlor-5,6-dicyano-1,4-benzochinon (= DDQ) versetzt und unter Rühren auf 50°C erwärmt. Nach 3h wurde das Solvens im Vakuum entfernt, der Rückstand mehrfach mit einem 4:1-Gemisch aus Cyclohexan und Ethylacetat extrahiert. Die organischen Extrakte wurden erneut eingedampft. Die Reinigung des Rückstands erfolgte durch Filtration über Kieselgel (Cyclohexan/Ethylacetat 4:1).

Ausbeute: 19.95 g XV (82 %) als weiße Kristalle
Rf-Wert (Cyclohexan/Ethylacetat 4:1) 0.73
Fp: 105°C
$^1$H-NMR: δ/ppm = 1.1 (t, J = 7Hz,3H), 1.4 (d,J = 7Hz,6H), 3.2 (h, J = 7Hz, 1H),4.2 (4, J = 7Hz, 2H), 7.0-8.0 (m,7H), 8.5-8.8 (m,2H).

Beispiele 4a - 4b

Die Verbindungen XVa und XVb wurden in analoger Weise, wie in Versuchsvorschrift beschrieben, dargestellt (vgl. Tabelle 6).

Tabelle 6

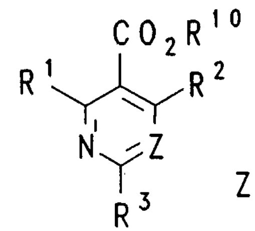

Z = N

| Bsp. | Verb. | $R^1$ | $R^2$ | $R^3$ | $R^{10}$ | Ausb. % | $R_f$-Wert Fp°C | $^1$H-NMR: $\delta$/ppm = MS : m/e = |
|---|---|---|---|---|---|---|---|---|
| a | XVa | $iC_3H_7$ | $4\text{-}FC_6H_4$ | $iC_3H_7$ | $C_2H_5$ | 79 | 0.87[c] Öl | 1.1 (t,3H), 1.25 (d,6H); 1.4 (d,6H), 3.2 (H,2H), 4.2 (q,2H), 7.0-7.9 (m,4H) 330 ($M^+$) |
| b | XVb | $iC_3H_7$ | $4\text{-}FC_6H_4$ | $4\text{-}FC_6H_4$ | $C_2H_5$ | 66,5 | 0.58[c] Öl | 386 ($M^+$) |

[a]Dichlormethan/Methanol 9:1 [b]Cyclohexan/Ethylacetat 4:1 [c]Cyclohexan/Ethylacetat 2:1

Beispiel 5

Allgemeine Vorschrift zur Herstellung der Verbindungen der allgemeinen Formel XVI

Versuchsvorschrift 5 ($R^1 = CH_3$, $R^2 = 4\text{-}FC_6H_4$, $R^3 = CH_3$, Z = CH) 2,6-Dimethyl-4-(4-fluorphenyl)pyridin-3-yl-methanol XVI

Eine Lösung von 7.8 g (30.1 mmol) 2,6-Dimethyl-4-(4-fluorphenyl)-pyridin-3-carbonsäuremethylester XV (aus Beispiel 2) in 40 ml Tetrahydrofuran wurde unter Feuchtigkeitsausschluß tropfenweise mit 30 ml (30 mmol) einer 1.0 M Lösung von Lithiumaluminiumhydrid in Tetrahydrofuran versetzt. Nach 1.5 stündigem Nachrühren wurde das Gemisch auf Wasser gegeben, mit Ether mehrfach extrahiert, mit Wasser gewaschen, über $MgSO_4$ getrocknet und eingedampft. Das zurückbleibende, rohe XVI wurde mit einem 1:1-Gemisch aus Cyclohexan und Ethylacetat gewaschen.

Ausbeute: 6.5 g (93%)

Fp.: 124°C

$^1$H-NMR ($CDCl_3$; 60 MHz)

$\delta$ = 2.0 (s,1H); 2.5 (s,3H); 2.7 (s,3H); 4.6 (s,2H); 6.9 (s,1H); 7.0-7.5 (m,4H) ppm

MS: m/e = 231 ($M^+$)

Beispiel 5a - 5k

Die Verbindungen XVIa - XVIk wurden in analoger Weise wie in Versuchsvorschrift 5 beschrieben, dargestellt (vgl. Tabelle 7)

Tabelle 7

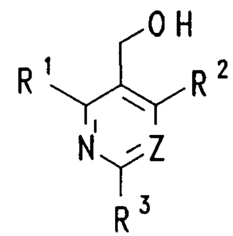

| Bsp. | Verb. | Z | $R^1$ | $R^2$ | $R^3$ | Ausb. % | $R_f$-Wert<br>Fp°C | $^1$H-NMR: $\delta$/ppm =<br>MS : m/e = |
|---|---|---|---|---|---|---|---|---|
| a | XVIa | CH | $iC_3H_7$ | $4\text{-}FC_6H_4$ | $C_6H_5$ | 69 | 0.33[b]<br>176 | 1.4 (d,J=7Hz,6H), 1.5 (brs,1H), 3.6 (h,J=7Hz,1H), 4.5 (s,2H), 7.0-7.6 (m,8H), 8.0-8.2 (m,2H).<br>321 ($M^+$) |
| b | XVIb | CH | $4\text{-}FC_6H_4$ | $iC_3H_7$ | $C_6H_5$ | 79 | 0.20[b]<br>157 | 1.4 (d,J=7Hz,6H), 1.7 (brs,1H), 3.5 (h,J=7Hz,1H), 4.7 (s,2H), 7.0-8.2 (m,10H)<br>322 ($M^+$+H) |

| Bsp. | Verb. | Z | $R^1$ | $R^2$ | $R^3$ | Ausb. % | $R_f$-Wert Fp°C | $^1$H-NMR: δ/ppm = MS : m/e = |
|---|---|---|---|---|---|---|---|---|
| c | XVIc | CH | $tC_4H_9$ | $4\text{-}FC_6H_4$ | $C_6H_5$ | 88 | $0.40^b$ Öl | 1.4 (s,9H), 4.6 (s,2H), 7.0-7.6 (m,8H), 8.1-8.3 (m2H) 336 ($M^+$ +H) |
| d | XVId | CH | $iC_3H_7$ | $4\text{-}CH_3OC_6H_4$ | $C_6H_5$ | 93 | $0.26^b$ 173-175 | 1.4 (d,J=7Hz,6H), 1.5 (s,1H), 3.6 (h,J=7Hz,1H), 3.9 (s.3H), 4.7 (s, 2H), 7.0-7.6 (m,8H), 8.1-8.3 (m,2H) 334 ($M^+$ +H) |
| e | XVIe | CH | $iC_3H_7$ | $4\text{-}FC_6H_4$ | $2,5\text{-}(CH_3)_2\text{-}C_6H_3$ | 89 | $0.48^b$ Öl | 1.4 (d,J=7Hz,6H), 1.6 (brs,1H), 2.3 (s,3H), 2.4 (2,3H), 3.6 (h, J=7Hz,1H), 4.7 (s,2H), 7.0-7.6 (m,8H) 350 ($M^+$ +H) |
| f | XVIf | CH | $iC_3H_7$ | $4\text{-}FC_6H_4$ | $4\text{-}FC_6H_4$ | 92 | $0.40^b$ 174-176 | 1.4 (d,J=7Hz,6H), 1.6 (brs,1H), 3.6 (h,J=7Hz,1H), 4.8 (s,2H), 7.0-7.6 (m,7H), 8.0-8.3 (m,2H) 340 ($M^+$ +H) |

| Bsp. | Verb. | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|
| g | XVIg | CH | $iC_3H_7$ | $4\text{-}FC_6H_4$ | $iC_3H_7$ | 91 | $0.56^b$ 90 | 1.3 (d,J=7Hz,6H), 1.4 (d,J=7Hz, 6H), 1.5 (s,1H), 3.1 (h,J=7Hz, 1H), 3.5 (h,J=7Hz,1H), 4.6 (s, 2H), 6.9-7.5 (m,5H) 288 ($M^+$+H) |
| h | XVIh | CH | $iC_3H_7$ | $4\text{-}FC_6H_4$ | $tC_6H_9$ | 95 | $0.36^d$ Öl | 1.3 (d,J=7Hz,6H), 1.4 (s,9H), 1.6 (s,1H), 3.4 (h,J=7Hz,1H), 4.6 (s, 2H), 7.0 (s,1H), 7.1-7.6 (m,4H) 301 ($M^+$) |
| i | XVIi | CH | $cC_6H_{11}$ | $4\text{-}FC_6H_4$ | $C_6H_5$ | 78 | $0.4^b$ 181-183 | 1.0-2.1 (m,11H), 2.9-3.5 (m,1H), 4.6 (s,2H), 7.0-7.6 (m,8H), 8.0-8.3 (m,2H) 361 ($M^+$) |
| j | XVIj | N | $iC_3H_7$ | $4\text{-}FC_6H_4$ | $iC_3H_7$ | 81 | $0.47^b$ Öl | 4.6 (s,2H), 1.6 (s,1H) |

| Bsp. | Verb. | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|
| k | XVIk | N | $iC_3H_7$ | $4\text{-}FC_6H_4$ | $4\text{-}FC_6H_4$ | 58 | 0.40[b] 117 | - 340($M^+$) |

[a]Cyclohexan/Ethylacetat 1:1 [b]Cyclohexan/Ethylacetat 4:1 [c]Dichlormethan/Methanol 9:1
[d]Cyclohexan/Ethylacetat 10:1

Beispiel 6

Allgemeine Vorschrift zur Herstellung der Verbindungen der allgemeinen Formel XVII

Versuchsvorschrift 5 ($R^1$ = $CH_3$, $R^2$ = 4-$FC_6H_4$ $R^3$ = $CH_3$, Z = CH, X-Br) 2,6-Dimethyl-4-(4-fluorphenyl)pyridin-3-yl)methylenbromid XVII
Eine Lösung von 6.4 g (27.7 mmol) 2,6-Dimethyl-4-(4-fluorphenyl)pyridin-3-ylmethanol XVI in einem Solvensgemisch aus 50 ml Toluol und 25 ml Dichlormethan wurde tropfenweise mit 5.3 ml (54.4 mmol) phosphortribromid versetzt und 1 h bei Raumtemperatur gerührt. Der Ansatz wurde auf gesättigte $NaHCO_3$-Lösung gegeben und mehrfach mit Ether ausgeschüttelt. Die vereinigten organischen Extrakte wurden mit Wasser gewaschen; über MgSO4 getrocknet und eingedampft. Zurück blieben 6.4 g (79 %) XVII, das ohne Reinigung weiter umgesetzt werden konnte.
Fp.: 86-87°C

$^1$H-NMR ($CDCl_3$; 60 MHz):
$\delta$ = 2.5 (s,3H); 2.7 (s,3H); 4.4 (s,2H); 6.9 (s,1H); 7.0-7.5 (m,4H) ppm.

MS: m/e = 295, 293 ($M^+$)

Beispiel 6a - 6m

Die Verbindungen XVIIa - XVIIm wurden in analoger Weise wie in Versuchsvorschrift 6 beschrieben, dargestellt (vgl. Tabelle 8)

Tabelle 8

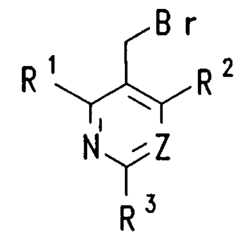

| Bsp. | Verb. | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|
| a | XVIIa | CH | $iC_3H_7$ | $4\text{-}FC_6H_4$ | $C_6H_5$ | 73 | $0.86^b$ Öl | 1.3 (d,J=7Hz,6H), 3.5 (h,J=7Hz, 1H), 4.4 (2,2H), 7.0-7.6 (m,8H), 8.0-8.2 (m,2H). 385,383 ($M^+$) |
| b | XVIIb | CH | $4\text{-}FC_6H_4$ | $iC_3H_7$ | $C_6H_5$ | 69 | $0.72^b$ 122 | 1.4 (d,J=7Hz,6H), 3.5 (h,J=7Hz, 1H), 4.6 (s,2H), 7.0-8.2 (m,10H) 386,384 ($M^+$+H) |
| c | XVIIc | CH | $tC_4H_9$ | $4\text{-}FC_6H_4$ | $C_6H_5$ | 83 | $0.84^b$ Öl | 1.4 (s,9H), 4.2 (s,2H), 7.0-7.6 (m,8H), 8.0-8.2 (m,2H) 400,398 ($M^+$+H) |

| Bsp. | Verb. | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|
| d | XVIId | CH | $iC_3H_7$ | $4CH_3OC_6H_4$ | $C_6H_5$ | 78 | 0.75[b] 103-105 | 1.5 (d,J=7Hz,6H), 3.6 (h,J=7Hz, 1H), 3.9 (s.3H), 4.5 (s,2H), 7.0-7.6 (m,8H), 8.1-8.3 (m,2H) 398,396 ($M^+$+H) |
| e | XVIIe | CH | $iC_3H_7$ | $4FC_6H_4$ | 2,5-$(CH_3)_2$-$C_6H_3$ | 71 | 0.80[b] Öl | 1.3 (d,J=7Hz,6H), 2.3 (s,3H), 2.4 (2,3H), 3.5 (h,J=7Hz,1H), 4.4 (s,2H), 6.9-7.5 (m,8H) 414,412 ($M^+$+H) |
| f | XVIIf | CH | $iC_3H_7$ | 4-$FC_6H_4$ | 4-$FC_6H_4$ | 89 | 0.87[b] 111-113 | 1.4 (d,J=7Hz,6H), 3.5 (h,J=7Hz, 1H), 4.5 (s,2H), 7.0-7.6 (m,7H), 8.0-8.2 (m,2H) 401,399 ($M^+$+H) |
| g | XVIIg | N | $iC_3H_7$ | 4-$FC_6H_4$ | $C_6H_5$ | 84 | 0.73[b] 134 | 1.4 (d,J=7Hz,6H), 3.6 (h,J=7Hz, 1H), 4.6 (s,2H), 7.1-8.6 (m,9H) 386,384 ($M^+$+H) |

| Bsp. | Verb. | Z | $R^1$ | $R^2$ | $R^3$ | Ausb. % | $R_f$-Wert Fp°C | $^1$H-NMR: δ/ppm = MS : m/e = |
|---|---|---|---|---|---|---|---|---|
| h | XVIIh | CH | $iC_3H_7$ | $4\text{-}FC_6H_4$ | $iC_6H_4$ | 86 | $0.95^d$ 81 | 1.3 (d,J=7Hz,6H), 1.4 (d,J=7Hz, 6H), 3.1 (h,J=7Hz,1H), 3.5 (h, J=7Hz,1H), 4.5 (s,2H), 6.9 (s,1H), 7.0-7.6 (m,4H) 352,350 ($M^+$) |
| i | XVIIi | CH | $iC_3H_7$ | $4\text{-}FC_6H_4$ | $tC_4H_9$ | 90 | $0.87^e$ Öl | 1.3 (s,9H), 1.3 (d,J=7Hz,6H), 3.5 (h,J=7Hz,1H), 4.4 (s,2H), 7.0-7.6 (m,5H) 365, 363 ($M^+$) |
| j | XVIIj | CH | $iC_3H_7$ | $4\text{-}FC_6H_4$ | $cC_6H_{11}$ | 86 | $0.89^e$ 84-86 | 1.4 (d,J=7Hz,6H), 1.4-2.1 (m, 10H), 2.7 (mc,1H), 3.5 (h,J=7Hz, 1H), 4.5 (s,2H), 6.9-7.6 (m,5H) 391,389 ($M^+$) |
| k | XVIIk | CH | $cC_6H_{11}$ | $4\text{-}FC_6H_4$ | $C_6H_5$ | 93 | $0.53^c$ 92-94 | 1.2-2.3 (m,11H), 3.2 (mc,1H), 4.5 (s,2H), 7.1-7.7 (m,7H), 8.0-8.2 (m,2H) 426, 424 ($M^+$+H) |

| Bsp. | Verb. | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|
| l | XVIII | N | $iC_3H_7$ | $4\text{-}FC_6H_4$ | $iC_3H_7$ | 83 | $0.63^b$ Öl | 1.25 (d,6H), 1.40 (d,6H), 3.4 (h, 2H), 4.48 (s,2H), 7.0-8.0 (m,4H) 350 ($M^+$ +H) |
| m | XVII m | N | $iC_3H_7$ | $4\text{-}FC_6H_4$ | $iC_3H_7$ | 71 | $0.65^b$ Öl | - 404 ($M^+$ +H) |

[a]Cyclohexan/Ethylacetat 1:1 [b]Cyclohexan/Ethylacetat 2:1 [c]Cyclohexan/Ethylacetat 4:1
[d]Dichlormethan/Methanol 9:1 [e]Cyclohexan/Ethylacetat 10:1

Beispiel 7

Allgemeine Vorschrift zur Herstellung der Verbindungen der allgemeinen Formel III

Versuchsvorschrift 7 ($R^1 = CH_3$, $R^2$ = 4-$FC_6H_4$, $R^3 = CH_3$, Z = CH, X = Br) 2,6-Dimethyl-4-(4-fluorphenyl)pyridin-3-yl)methyltriphenyl-phosphoniumbromid III
Eine Lösung von 6.4 g (22.5 mmol) 2,6-Dimethyl-4-(4-fluorphenyl)pyridin-3-yl-methylenbromid XVII und 6.2 g (23 mmol) Triphenylphosphan in 200 ml Toluol wurde unter DC-Kontrolle 5h lang refluxiert. Beim Abkühlen der Reaktionslösung fiel das gebildete Phosphoniumsalz in Form weißer Kristalle aus. Diese wurden abgesaugt, mit Ether gewaschen und im Vakuum getrocknet. Die Ausbeute an III betrug 11.2 g (89%).
Fp.: 218 - 220°C

$^1$H-NMR($CDCl_3$; 60MHz):
$\delta$ = 2.3 (d, J = 2Hz,3H); 2.5 (d,J = 3Hz,3H); 6.3 (d, J = 16Hz,2H); 6.8-7.9 (m,20H) ppm.

MS: 476 ($M^+$ Kation)

Beispiel 7a - 7m

Die Verbindungen IIIa - IIIm wurden in analoger Weise, wie in Versuchsvorschrift 7 beschrieben, dargestellt (vgl.Tabelle 9).

Tabelle 9

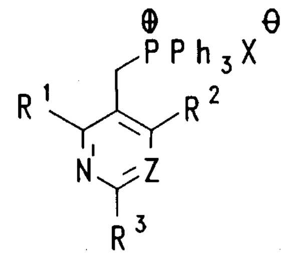

| Bsp. | Verb. | X | Z | $R^1$ | $R^2$ | $R^3$ | Ausb. % | $R_f$-Wert Fp°C | $^1$H-NMR: δ/ppm = MS : m/e = |
|---|---|---|---|---|---|---|---|---|---|
| a | IIIa | Br | CH | $iC_3H_7$ | $4-FC_6H_4$ | $C_6H_5$ | 68 | $0.06^b$ 250 | 1.0 (d,J=7Hz,6H), 3.0 (mc, 1H), 5.6 (d,J=14Hz,2H), 6.8-8.2 (m,25H) 566 ($M^+$ Kation) |
| b | IIIb | Br | CH | $4-FC_6H_4$ | $iC_3H_7$ | $C_6H_5$ | 75 | $0.25^c$ 254 | 0.9 (d,J=7Hz,6H), 3.4 (mc, 1H), 5.2 (d,J=14Hz,2H), 7.0-8.2 (m,25H) 566 ($M^+$ Kation) |

| Bsp. | Verb. | X | Z | $R^1$ | $R^2$ | $R^3$ | Ausb. % | $R_f$-Wert Fp°C | $^1$H-NMR: δ/ppm = MS : m/e = |
|---|---|---|---|---|---|---|---|---|---|
| c | IIIc | Br | CH | t-$C_4H_9$ | 4-$C_4H_9$ | $C_6H_5$ | 71 | 0.08[b] 250 | 1.0 (s,9H), 5.8 (d,J=14Hz, 2H), 6.8-8.2 (25H) 580 ($M^+$ Kation) |
| d | IIId | Br | CH | i$C_3H_7$ | 4$CH_3OC_6H_4$ | $C_6H_5$ | 92 | 0.05[b] 274 | 1.0 (d,J=7Hz,6H), 3.0 (mc, 1H), 3.9 (s,3H), 6.5 (d, J=14Hz,2H), 6.8-8.2 (m,25H) 578 ($M^+$ Kation) |
| e | IIIe | Br | CH | i$C_3H_7$ | 4-$FC_6H_4$ | 2,5$(CH_3)_2$-$C_6H_3$ | 86 | 0.06[b] 250 | 0.8 (d,J=7Hz,6H), 2.3 (s,6H), 3.8 (d,J=7Hz,1H), 5.0 (d, J=14Hz,2H), 7.0-7.9 (m,23H) 7.0-7.9 (m,23H) 594 ($M^+$ Kation) |
| f | IIIf | Br | CH | i$C_3H_7$ | 4-$FC_6H_4$ | 4-$FC_6H_4$ | 91 | 0.04[b] 95-98 | 1.0 (d,J=7Hz,6H), 3.0 (mc, 1H), 5.5 (d,J=14Hz,2H), 7.0-8.2 (m,24H) 584 ($M^+$ Kation) |

| Bsp. | Verb. | X | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|---|
| g | IIIg | Br | N | $iC_3H_7$ | $4\text{-}FC_6H_4$ | $C_6H_5$ | 94 | $0.52^c$ 272-274 | - 567 ($M^+$ Kation) |
| h | IIIh | CH | Br | $iC_3H_7$ | $4\text{-}FC_6H_4$ | $iC_3H_7$ | 80 | $0.02^b$ 110 (Zers.) | 0.9 (d,J=7HZ,6H), 1.2 (d, J=7Hz,6H), 2.6-3.2 (m,2H), 5.4 (d,J=16Hz,2H), 6.7-7.9 (m,20H), 532 ($M^+$ Kation) |
| i | IIIi | CH | Br | $iC_3H_7$ | $4\text{-}FC_6H_4$ | $tC_4H_9$ | 74 | - >100 (Zers.) | 0.9 (d,J=7Hz,6H), 1.3 (s,9H), 2.9 (mc,1H), 5.5 (d,J=14Hz, 2H), 6.8-7.9 (m,20H) 546 ($M^+$ Kation) |
| j | IIIj | CH | Br | $iC_3H_7$ | $4\text{-}FC_6H_4$ | $cC_6H_{11}$ | 98 | - 223-226 (Zers.) | 0.9 (d,J=7Hz,6H), 1.4-2.0 (m,10H), 2.2-3.0 (m,2H), 5.5 (d,J=14Hz,2H), 6.8-7.6 (m, 20H) 572 ($M^+$ Kation) |

| Bsp. | Verb. | X | Z | $R^1$ | $R^2$ | $R^3$ | Ausb. % | $R_f$-Wert Fp°C | $^1$H-NMR: $\delta$/ppm = MS : m/e = |
|---|---|---|---|---|---|---|---|---|---|
| k | IIIk | CH | Br | $cC_6H_{11}$ | $4\text{-}FC_6H_4$ | $C_6H_4$ | 97 | - >270 (Zers.) | 0.8-2.0 (m,11H), 2.5 (mc,1H), 5.5 (d,J=16Hz,2H), 7.0-8.2 (m,25H) 606 ($M^+$ Kation) |
| l | IIIl | N | Br | $iC_3H_7$ | $4\text{-}FC_6H_4$ | $iC_3H_7$ | 59 | - >250 (Zers.) | 0.95 (d,6H), 1.4 (d,6H), 3.15 (h,2H), 5.8 (d,J=16Hz,2H), 7.0-8.0 (m,19H), 533 ($M^+$ Kation) |
| m | IIIm | N | Br | $iC_3H_7$ | $4\text{-}FC_6H_4$ | $4\text{-}FC_6H_4$ | 78 | - 248-255 | 1.0 (d,6H), 3.0 (h,1H), 5.6 (d, J=16Hz,2H), 7.0-8.8 (m, 23H) |

[a]Cyclohexan/Ethylacetat 1:1 [b]Cyclohexan/Ethylacetat 2:1 [c]Dichlormethan/Methanol 9:1

Beispiel 8

Allgemeine Vorschrift zur Herstellung der Verbindungen der allgemeinen Formel V

Versuchsvorschrift 8 ($R^1$ = $CH_3$, $R^2$ = 4-$FC_6H_4$, $R^3$ = $CH_3$, A-B = -CH = CH-) (4R,6S)-4-(tert.Butyldiphenylsilyloxy)-6-(2-(2,6-dimethyl-4-(4-fluorphenyl)-pyridin-3-yl)ethenyl)-2-methoxy-3,4,5,6-tetrahydropyranone Z-V und E-V
Zu einer Lösung von 9.70 g (17.5 mmol) 2,6-Dimethyl4-(4-fluorphenyl)pyridin-3-yl)methyltriphenylphosphoniumbromid III in 100 ml THF wurden bei 0°C 12.0 ml (19.2 mmol) einer 1.6 molaren Lösung von n-Butyllithium in Hexan getropft. Das Gemisch wurde 30 min bei Raumtemperatur gerührt und anschließend tropfenweise mit einer Lösung von 7.29 g (18.4 mmol) des Aldehyds IV in 40 ml THF versetzt. Nach einstündigem Rühren wurde das Gemisch durch Zusatz von Wasser abgeschreckt, mit Essigsäure auf einen pH-Wert zwischen 5 und 6 gebracht und mehrfach mit Ether extrahiert. Die vereinigten organischen Extrakte wurden mit gesättigter Natriumhydrogencarbonatlösung und Wasser gewaschen, über Magnesiumsulfat getrocknet und eingedampft. Das zurückbleibende rohe Diastereomerengemisch wurde säulenchromatographisch (Kieselgel, Cyclohexan/Ethylacetat 2:1) aufgetrennt.
Ausbeute: 2.36 g Z-V, sowie 4.99 g E-V, entsprechend 70 % bei einem
Z:E-Verhältnis von 32:68 (ca. 1:2).
Z-Va: Fp: 111-113°C

$^1$H-NMR: δ/ppm = 0.9 (s,9H), 1.0-1.8 (m,4H), 2.6 (s,6H), 3.3 (s,3H), 4.2 (mc,1H), 4.3 (mc,1H), 5.5 (mc,1H), 6.3 (d, J = 10Hz, 1H), 6.9-7.8 (m,15H).

MS: m/e = 596 ($M^+$ H)
E-Va: Fp:Öl

$^1$H-NMR: δ/ppm = 1.1 (s,9H), 1.1-1.9 (m,4H), 2.5 (s,3H), 2.6 (s,3H), 3.5 (s,3H), 4.2 (mc,1H), 4.5 (mc,1H), 4.9 (mc,1H), 5.5 (dd, J = 16Hz,6Hz,1H), 6.43 (d, J = 16Hz,1H), 6.9-7.7 (m,15H)

MS: m/e = 596 ($M^+$ H)

Beispiel 8a - 8m

Die Verbindungen Va - Vm wurden in analoger Weise, wie in Versuchsvorschrift 8 beschrieben, dargestellt (vgl. Tabelle 10)

Tabelle 10

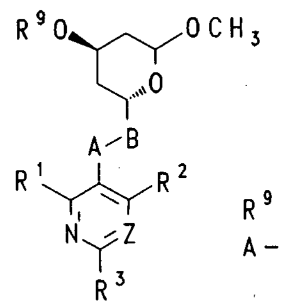

$R^9 = Si(C_6H_5)_2 tC_4H_9$
$A-B = -CH=CH-$

| Bsp. | Verb. | Z | $R^1$ | $R^2$ | $R^3$ | Ausb. % Z:E | $R_f$-Wert (Z;E) Fp°C (Z;E) | $^1$H-NMR: $\delta$/ppm = MS : m/e = |
|---|---|---|---|---|---|---|---|---|
| a | Va | CH | $iC_3H_7$ | $4-FC_6H_4$ | $C_6H_5$ | 74 <1:>20 | -; 0.89[a] | E: 1.1 (s,9H), 1.3-1.9 (m,10H), 3.4-3.5 (m,4H), 4.3 (mc,1H), 4.9 (mc, 1H), 6.5 (d,J=16Hz, 1H), 7.0 (mc, 2H), 7.2 (mc,2H), 7.2-7.7 (m,12H), 8.1 (mc,4H) |
| b | Vb | CH | $4FC_6H_4$ | $iC_3H_7$ | $C_6H_5$ | <1:>20 | -; 0.62[d] -; Öl | 685 ($M^+$) |

| Bsp. | Verb. | Z | $R^1$ | $R^2$ | $R^3$ | Ausb. % Z:E | $R_f$-Wert (Z;E) Fp °C (Z;E) | $^1$H-NMR: $\delta$/ppm = MS : m/e = |
|---|---|---|---|---|---|---|---|---|
| c | Vc | CH | $tC_4H_9$ | $4\text{-}FC_6H_4$ | $C_6H_5$ | <1:>20 | -; 0.70[d] -; | 699 ($M^+$) |
| d | Vd | CH | $iC_3H_7$ | $4\text{-}CH_3OC_6H_4$ | $C_6H_5$ | 69 <1:>20 | -; 0.64[d] -; Öl | 690 ($M^+$+H) |
| e | Ve | CH | $iC_3H_7$ | $4\text{-}FC_6H_4$ | $2,5\text{-}(CH_3)_2\text{-}C_6H_3$ | 72 <1:>20 | -; 0.77[d] -; Öl | 1.1 (s,9H), 1.2-1.4 (m,9H), 1.9 (mc,1H), 2.4 (s,3H), 2.5 (s,3H), 3.4 (h,J=7Hz,1H), 3.5 (s,3H), 4.3 (mc, 1H), 4.5 (mc,1H), 4.9 (mc,1H), 5.4 (mc,1H), 6.6 (d,J=16Hz,1H), 7.0-7.5 (m,14H), 7.7 (mc,4H) 714 ($M^+$+H) |
| f | Vf | CH | $iC_3H_7$ | $4\text{-}FC_6H_4$ | $iC_3H_7$ | 76 <1:>20 | -; 0.56[d] -; Öl | 704 ($M^+$+H) |

| Bsp. | Verb. | Z | $R^1$ | $R^2$ | $R^3$ | Ausb. % Z:E | $R_f$-Wert (Z;E) Fp°C (Z;E) | $^1$H-NMR: $\delta$/ppm = MS : m/e = |
|---|---|---|---|---|---|---|---|---|
| g | Vg | N | $iC_3H_7$ | $4\text{-}FC_6H_4$ | $C_6H_5$ | 73 2:1 | 0.27; 0.44[d] | E: 1.1 (s,9H), 1.1-1.9 (m,10H), 3.4 (h,J=7Hz,1H), 3.5 (s,3H), 4.3 (mc, 1H), 4.9 (mc,1H), 5.6 (mc,1H), 6.6 (dd,J=16Hz,1Hz,1H), 7.1-7.8 (m, 17H), 8.5-8.6 (m,2H) |
| h | Vh | CH | $iC_3H_7$ | $4\text{-}FC_6H_4$ | $iC_3H_7$ | 62 <1:>20 | -; 0.88[b] -; Öl | 1.1 (s,9H), 1.2-1.8 (m,16H), 3.0 (h,J=7Hz,1H), 3.4 (h,J=7Hz,1H), 3.5 (s,3H), 4.2 (mc,1H), 4.5 (mc, 1H), 4.9 (mc,1H), 5.3 (mc,1H), 6.5 (dd,J=16Hz,2Hz,1H), 6.9-7.7 (m, 15H), |
| i | Vi | CH | $iC_3H_7$ | $4\text{-}FC_6H_4$ | $tC_4H_9$ | 84 <1:>20 | -; 0.58[e] -; Öl | 1.1 (s,9H), 1.3 (mc,6H), 1.4 (s,9H), 1.5-1.9 (m,4H), 3.4 (h,J=7Hz,1H), 3.5 (s,3H), 4.2 (mc,1H), 4.5 (mc, 1H), 4.9 (mc,1H), 5.4 (dd,J=16Hz, 6Hz,1H), 6.5 (dd,J=16Hz,1Hz,1H), 6.9-7.7 (m,15H), 666 ($M^+$+H) |

| Bsp. | Verb. | Z | $R^1$ | $R^2$ | $R^3$ | Ausb. % Z:E | $R_f$-Wert (Z;E) Fp°C (Z;E) | $^1$H-NMR: δ/ppm = MS : m/e = |
|---|---|---|---|---|---|---|---|---|
| j | Vj | CH | $iC_3H_7$ | $4\text{-}FC_6H_4$ | $cC_6H_{11}$ | 49<br><br><1:>20 | -; 0.40[f]<br><br>-; Öl | 1.1 (s,9H), 1.3 (mc,6H), 1.4-2.0 (m,14H), 2.7 (mc,1H), 3.3 (h, J=7Hz,1H), 3.5 (s,3H), 4.2 (mc, 1H), 4.5 (mc, 1H), 5.3 (mc,1H), 6.5 (d,J=16Hz,1H), 6.7-7.7 (m,15H),<br>692 ($M^+$+H) |
| k | Vk | CH | $cC_6H_{11}$ | $4\text{-}FC_6H_4$ | $C_6H_5$ | 50 | -; 0.50[b]<br><br>-; Öl | E: 1.1 (s,9H), 1.3-2.0 (m,14H), 3.5 (s,3H), 4.2 (mc, 1H), 4.5 (mc,1H), 5.4 (mc,1H), 6.5 (dd,J=16Hz,1Hz, 1H), 7.0-7.5 (m, 12H), 8.0-8.2 (m,3H)<br>726 ($M^+$+H) |
| l | Vl | N | $iC_3H_7$ | $4\text{-}FC_6H_4$ | $iC_3H_7$ | 89 | -; 0.70[a]<br>-; Öl | 3.5 (s,3H), 6.5 (dd,J=16Hz,1H)<br>653 ($M^+$+H) |

| Bsp. | Verb. | Z | $R^1$ | $R^2$ | $R^3$ | Ausb. % Z:E | $R_f$-Wert (Z;E) Fp°C (Z;E) | $^1$H-NMR: $\delta$/ppm = MS : m/e = |
|---|---|---|---|---|---|---|---|---|
| m | Vm | N | $iC_3H_7$ | 4-$FC_6H_4$ | 4-$FC_6H_4$ | 67 | -; 0.64[a] -; Öl | 705 ($M^+$ +H) |

[a]Cyclohexan/Ethylacetat 2:1 [b]Cyclohexan/Ethylacetat 4:1 [c]Cyclohexan/Ethylacetat 8:1
[d]Cyclohexan/Ethylacetat 1:1 [e]Cyclohexan/Ethylacetat 10:1 [f]Cyclohexan/Ethylacetat 30:1

Beispiel 9

Allgemeine Vorschrift zur Herstellung der Verbindungen der allgemeinen Formel VI

Beispiel 9 ($R^1 = CH_3$, $R^2$ = 4-$FC_6H_4$, $R^3 = CH_3$, A-B = -CH = CH-)
(4R, 6S)-4-(tert.Butyldiphenylsilyloxy)-6-(2(2,6-dimethyl-4-(4-fluorphenyl) pyridin-3-yl)ethenyl)-2-hydroxy-3,4,5,6-tetrahydro-2H-pyrane Z-VI und E-VI
7.25 g (12.4 mmol) eines 32:68-Gemisches der Verbindungen Z-V und E-V wurden in einem Solvensgemisch aus 85 ml THF, 85 ml Wasser und 140 ml Eisessig 48 h unter Rückfluß gekocht. Nach Zusatz von 200 ml Toluol wurde die Reaktionslösung eingedampft. Durch mehrfaches Eindampfen unter Zusatz von Toluol wurde der Rückstand von Essigsäureresten befreit, anschließend in Ether aufgenommen, mit gesättigter Natriumhydrogencarbonatlösung geschüttelt, über Magnesiumsulfat getrocknet und erneut eingedampft. Eine anschließende chromatographische Reinigung lieferte 1.35 g Z-VI, sowie 3.14 9 E-VI, entsprechend einer Ausbeute von 63 % und einem Z:E-Verhältnis von 30:70.

| | |
|---|---|
| Z-VI | : Fp:147-149°C |
| $^1$H-NMR | : δ/ppm = 0.9 (s,9H), 1.0-1.9 (m,4H), 2.5 (s,6H), 4.0-4.4 (m,2H), 4.8-6.5 (m,3H), 6.9-7.6 (m,15H). |
| MS | : m/e = 581 ($M^+$) |
| E-VI | : Fp: 119°C |
| $^1$H-NMR | : δ/ppm = 1.1 (s,9H), 1.2-2.0 (m,4H), 2.5 (s,3H), 2.6 (s,3H), 3.9-5.0 (m,3H), 5.1-5.6 (m,2H), 6.4 (d,J = 16Hz, 1H), 6.9-7.8 (m, 15H). |
| MS | : m/e = 581 ($M^+$) |

Beispiel 9a-9m

Die Verbindungen VIa-VIm wurden in analoger Weise, wie in Versuchsvorschrift 9 beschrieben, dargestellt (vgl. Tabelle 11)

Tabelle 11

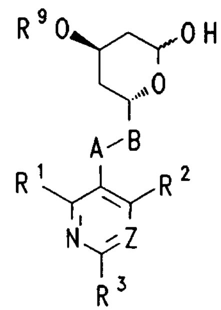

$R^9 = Si(C_6H_5)_2tC_4H_9$
$A-B = -CH=CH-$

| Bsp. | Verb. | Z | $R^1$ | $R^2$ | $R^3$ | Ausb. % | $R_f$-Wert (Z;E)<br>Fp°C (Z;E) | $^1$H-NMR: $\delta$/ppm =<br>MS : m/e = |
|---|---|---|---|---|---|---|---|---|
| a | VIa | CH | $iC_3H_7$ | $4-FC_6H_4$ | $C_6H_5$ | 74 | -; 0.32[c]<br>-; Öl | E: 1.1-1.8 (m,19H), 3.4 (h,J=7Hz, 1H), 4.3 (mc,1H), 4.9 (mc,1H), 5.2-5.6 (m,3H), 6.5 (mc,1H), 6.9-8.2 (m,20H)<br>671 ($M^+$) |
| b | VIb | CH | $4FC_6H_4$ | $iC_3H_7$ | $C_6H_5$ | 84 | -; 0.25[b]<br>-; Öl | 671 ($M^+$) |
| c | VIc | CH | $tC_4H_9$ | $4-FC_6H_4$ | $C_6H_5$ | 71 | -; 0.40[b]<br>-; Öl | 685 ($M^+$) |

| Bsp. | Verb. | Z | $R^1$ | $R^2$ | $R^3$ | Ausb. % | $R_f$-Wert (Z;E) Fp°C (Z;E) | $^1$H-NMR: $\delta$/ppm = MS : m/e = |
|---|---|---|---|---|---|---|---|---|
| d | VId | CH | $iC_3H_7$ | $4-CH_3OC_6H_4$ | $C_6H_5$ | 65 <1:>20 | -; 0.37[c] -; Öl | 683 ($M^+$) |
| e | VIe | CH | $iC_3H_7$ | $4-FC_6H_4$ | $2,5-(CH_3)_2-C_6H_3$ | 69 | -; 0.47[c] -; Öl | 699 ($M^+$) |
| f | VIf | CH | $iC_3H_7$ | $4-FC_6H_4$ | $4-FC_6H_4$ | 75 | -; 0.35[c] -; Öl | 705 ($M^+$) |
| g | VIg | N | $iC_3H_7$ | $4-FC_6H_4$ | $C_6H_5$ | 84 | 0.17; 0.15[a] Öl; Öl | E: 1.1 (s,9H), 1.2-2.6 (m,4H), 3.5 (h,J=7Hz,1H), 4.3 (mc,1H), 4.9 (mc,1H), 5.5 (mc,1H), 6.5 (d, J=16Hz,1H), 6.9-8.6 (m, 19H), 673 ($M^+$+H) |
| h | VIh | CH | $iC_3H_7$ | $4-FC_6H_4$ | $iC_3H_7$ | 50 | -; 0.55[b] -; Öl | - 638 ($M^+$+H) |

| Bsp. | Verb. | Z | $R^1$ | $R^2$ | $R^3$ | Ausb. % | $R_f$-Wert (Z;E)<br>Fp°C (Z;E) | $^1$H-NMR: $\delta$/ppm =<br>MS : m/e = |
|---|---|---|---|---|---|---|---|---|
| i | VIi | CH | $iC_3H_7$ | $4\text{-}FC_6H_4$ | $tC_4H_9$ | 54 | -; 0.54[d]<br>-; Öl | -<br>652 ($M^+$+H) |
| j | VIj | CH | $iC_3H_7$ | $4\text{-}FC_6H_4$ | $cC_6H_{11}$ | 48 | -; 0.41[d]<br>-; Öl | -<br>678 ($M^+$+H) |
| k | VIk | CH | $cC_6H_{11}$ | $4\text{-}FC_6H_4$ | $C_6H_5$ | 54 | -; 0.33 (4:1)<br>-; Öl | -<br>712 ($M^+$+H) |
| l | VIl | N | $iC_3H_7$ | $4\text{-}FC_6H_4$ | $iC_3H_7$ | 100 | -; 0.54[e]<br>-; Öl | -<br>639 ($M^+$+H) |
| m | VIm | N | $iC_3H_7$ | $4\text{-}FC_6H_4$ | $4\text{-}FC_6H_4$ | 98 | -; 0.30[e]<br>-; Öl | -<br>691 ($M^+$+H) |

[a]Cyclohexan/Ethylacetat 1:1 [b]Cyclohexan/Ethylacetat 4:1 [c]Cyclohexan/Ethylacetat 5:1 [d]Cyclohexan/Ethylacetat 10:1
[e]Cyclohexan/Ethylacetat 2:1

Beispiel 10

Allgemeine Vorschrift zur Herstellung der allgemeinen Formel VII

Versuchsvorschrift 10 ($R^1 = CH_3$, $R^2 = 4\text{-}FC_6H_4$, $R^3 = CH_3$, AB =-CH = CH-)
(4R,6S)-4-(tert.Butyldiphenylsilyloxy)-6-(2-(2,6-Dimethyl- 4-(4-fluorophenyl)pyridin-3-yl)ethenyl)-3,4,5,6-tetrahydro-2H-pyran-2-one Z-VII und E-VII
Eine Lösung von 8.32 g (37.0 mmol) N-Jodsuccinimid und 2.73 g (7.4 mmol) Tetra-n- butylammoniumiodid in 100 ml Dichlormethan wurde tropfenweise mit 4.30 g (7.4 mmol) eines 30:70-Gemisches der Verbindungen Z-VI und E-VI gelöst in 20 ml Dichlormethan, versetzt. Der Ansatz wurde 2 h bei Raumtemperatur gerührt, anschließend auf Wasser gegeben und mehrfach ausgeethert. Die vereingten Extrakte wurden mit Natriumthiosulfatlösung entfärbt, mit Wasser gewaschen, über Magnesiumsulfat getrocknet und eingedampft. Der Rückstand wurde in Diisopropylether aufgenommen, filtriert, und das Filtrat erneut eingedampft. Durch säulenchromatographische Reinigung des zurückbleibenden Öls (Kieselgel, mit 10 % Wasser desaktiviert, Cyclohexan/Ethylacetat 1:1) wurden 0.52 g Z-VII sowie 2.45 g E-VII erhalten. Dies entspricht einer Ausbeute von 76 % bei einem Z:E-Verhältnis von 25:75.
Z-VIIa:Fp: 188°C

$^1$H-NMR : δ/ppm = 0.9 (s,9H), 1.3-1.7 (m,4H), 2.4 (mc,2H), 2.6 (s,6H), 4.2 (mc,1H), 5.0 (mc,1H), 5.6 (mc,1H), 6.5 (d,J = 11Hz,1H), 6.9-7.5 (m,15H).
MS : m/e = 580 ($M^+$ + H)
E-VII : Fp:Öl
$^1$H-NMR : δ/ppm = 2.1 (s,9H), 1.3-1.7 (m,2H), 2.4-2.6 (m,8H), 4.2 (mc,1H), 5.2 (mc,1H), 5.4 (mc,1H), 6.5 (d,J = 16Hz,1H), 6.9-7.7 (m,15H),
MS : m/e = 580 ($M^+$ + H).

Beispiel 10a-10m

Die Verbindungen VIIa-VIIm wurden in analoger Weise, wie in Versuchsvorschrift 10 beschrieben, dargestellt (vgl. Tabelle 12)

Tabelle 12

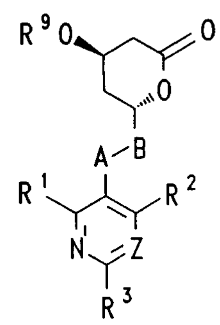

$R^9 = Si(C_6H_5)_2tC_4H_9$
$A-B = -CH=CH-$

| Bsp. | Verb. | Z | $R^1$ | $R^2$ | $R^3$ | Ausb. % | $R_f$-Wert (Z;E)<br>Fp°C (Z;E) | $^1$H-NMR: δ/ppm =<br>MS : m/e = |
|---|---|---|---|---|---|---|---|---|
| a | VIIa | CH | $iC_3H_7$ | $4-FC_6H_4$ | $C_6H_5$ | 70 | -; $0.51^b$<br>Öl; Öl | E: 1.1 (s,9H), 1.2-1.8 (m,10H), 3.4 (d,J=7Hz,1H), 4.3 (mc,1H), 5.2-5.4 (m,2H), 6.5 (d,J=16Hz,1H), 7.0-8.1 (m,20H)<br>669 ($M^+$) |
| b | VIIb | CH | $4FC_6H_4$ | $iC_3H_7$ | $C_6H_5$ | 76 | -; $0.36^b$<br>-; Öl | 669 ($M^+$) |

| Bsp. | Verb. | Z | $R^1$ | $R^2$ | $R^3$ | Ausb. % | $R_f$-Wert (Z;E)<br>Fp°C (Z;E) | $^1H$-NMR: $\delta$/ppm =<br>MS : m/e = |
|---|---|---|---|---|---|---|---|---|
| c | VIIc | CH | $tC_4H_9$ | $4-FC_6H_4$ | $C_6H_5$ | 83 | -; $0.45^b$<br>-; Öl | 682 ($M^+$) |
| d | VIId | CH | $iC_3H_7$ | $4-CH_3OC_6H_4$ | $C_6H_5$ | 88 | -; $0.55^b$<br>-; Öl | 681 ($M^+$) |
| e | VIIe | CH | $iC_3H_7$ | $4-FC_6H_4$ | $2,5-(CH_3)_2-C_6H_3$ | 79 | -; $0.60^b$<br>-; Öl | 697 ($M^+$) |
| f | VIIf | CH | $iC_3H_7$ | $4-FC_6H_4$ | $4-FC_6H_4$ | 65 | -; $0.54^d$<br>-; Öl | 687 ($M^+$) |
| g | VIIg | N | $iC_3H_7$ | $4-FC_6H_4$ | $C_6H_5$ | 93 | 0.39; $0.35^a$<br>Öl; Öl | 4.3 (mc,1H), 5.3 (mc,1H), 5.5 (mc, 1H), 6.7 (d,J=16Hz,1H), 7.0-7.7 (m,7H), 8.5-8.6 (m,2H)<br>656 ($M^+$) |

| Bsp. | Verb. | Z | $R^1$ | $R^2$ | $R^3$ | Ausb. % | $R_f$-Wert (Z;E)<br>Fp°C (Z;E) | $^1$H-NMR: $\delta$/ppm =<br>MS : m/e = |
|---|---|---|---|---|---|---|---|---|
| h | VIIh | CH | $iC_3H_7$ | $4\text{-}FC_6H_4$ | $iC_3H_7$ | 87 | -; $0.32^f$<br>-; Öl | -<br>636 ($M^+$ +H) |
| i | VIIi | CH | $iC_3H_7$ | $4\text{-}FC_6H_4$ | $tC_4H_9$ | 83 | -; $0.62^f$<br>-; Öl | 1.1 (s,9H), 1.3 (mc,6H), 1.4 (s,9H), 1.5-1.8 (m,2H), 2.4 (mc,1H), 2.6 (mc,1H), 3.3 (mc,1H), 4.2 (mc,1H), 5.2-5.3 (m,2H), 6.6 (d,J=16Hz, 1H), 7.0-7.7 (m,15H),<br>650 ($M^+$ +H) |
| j | VIIj | CH | $iC_3H_7$ | $4\text{-}FC_6H_4$ | $cC_6H_{11}$ | 93 | -; $0.56^f$<br>-; 90-92 | -<br>676 ($M^+$ +H) |

| Bsp. | Verb. | Z | $R^1$ | $R^2$ | $R^3$ | Ausb. % | $R_f$-Wert (Z;E)<br>Fp°C (Z;E) | $^1$H-NMR: $\delta$/ppm =<br>MS : m/e = |
|---|---|---|---|---|---|---|---|---|
| k | VIIk | CH | $cC_6H_{11}$ | $4-FC_6H_4$ | $C_6H_5$ | 91 | -; 0.41[e]<br>-; Öl | 1.1 (s,9H), 1.3-1.9 (m,12H), 2.5 (mc,1H), 2.6 (mc,1H), 3.0 (mc,1H), 4.2 (mc,1H), 5.2-5.4 (m,2H), 6.6 (d,J=16Hz,1H), 7.0 (mc,2H), 7.2-7.7 (m,21H), 8.1 (mc,2H)<br>709 ($M^+$+H) |
| l | VIII | N | $iC_3H_7$ | $4-FC_6H_4$ | $iC_3H_7$ | 88 | -; 0.37[e]<br>-; Öl | 1.1 (s,9H), 1.25 (d,6H), 1.48 (d,6H), 1.5-1.9 (m,2H), 2.1-2.7 (m,2H), 3.15-3.35 (m,2H), 4.25 (m,1H), 5.25 (m,1H), 5.4 (dd,1H), 6.6 (d,1H), 7.0 (t,1H), 7.35-7.6 (m,14H)<br>636 ($M^+$+H) |
| m | VIIm | N | $iC_3H_7$ | $4-FC_6H_4$ | $4-FC_6H_4$ | 97 | -; 0.35[e]<br>-; Öl | -<br>689 ($M^+$+H) |

[a]Cyclohexan/Ethylacetat 1:1 [b]Cyclohexan/Ethylacetat 3:1 [c]Cyclohexan/Ethylacetat 2:1 [d]Cyclohexan/Ethylacetat 20:1 [e]Cyclohexan/Ethylacetat 4:1 [f]Cyclohexan/Ethylacetat 10:1

Beispiel 11

Allgemeine Vorschrift zur Herstellung der Verbindungen der allgemeinen Formel I

Versuchsvorschrift 11 ($R^1$ = $CH_3$, $R^2$ = 4-$FC_6H_4$, $R^3$ = $CH_3$, A-B = -CH = CH-) (4R,6S)-6-(2-(2,6-Dimethyl-4-(4-fluorphenyl)pyridin-3-yl)ethenyl)-4-hydroxy-3,4,5, 6-tetrahydro-2H-pyran-2-one Z-I und E-I
Eine Lösung von 3.00 g (5.2 mmol) eines Gemisches der Verbindungen Z-VII und E-VII, 4.90 g (15.5 mmol) Tetra-n-butylammoniumfluoridtrihydrat und 11.8 ml (20.7 mmol) Essigsäure in 50 ml Tetrahydrofuran wurde 15 h bei Raumtemperatur gerührt. Anschließend wurde der Ansatz auf gesättigte Natriumhydrogencarbonatiösung gegeben und mehrfach ausgeethert. Die vereinigten organischen Extrakte wurden mit gesättigter Kochsalzlösung gewaschen, über Magnesiumsulfat getrocknet und eingedampft. Das zurückbleibende Produkt wurde chromatographiert (Kieselgel, desaktiviert mit 10% Wasser, Ethylacetat/Methanol 10:1).
Erhalten wurden 0.29 g des Z-Isomeren Z-I, sowie 0.97 g an E-I, gleichbedeutend mit einer Ausbeute von 71 % bei einem Diastereomerenverhältnis (Z:E) von 23:77.
Z-Ia:Fp: 188°C

$^1$H-NMR: δ/ppm = 1.5 (mc,1H), 1.8-2.2 (m,2H), 2.4-2.6 (m,8H), 4.2 (mc,1H), 4.8 (mc,1H), 5.6 (mc,1H), 6.5 (mc,1H), 6.9 (s,1H), 7.0-7.4 (m,4H),

MS:m/e = 341 ($M^+$)
E-I: Fp: 205°C

$^1$H-NMR: δ/ppm = 1.6-1.9 (m,3H), 2.5 (s,3H), 2.6 (s,3H), 2.6-2,8 (m,2H), 4.3 (mc,1H), 5.3 (mc,3H), 5.5 (mc,1H), 6.6 (d,J = 16Hz,1H), 6.9 (s,1H), 7.0-7.3 (m,4H)

MS: m/e = 341 ($M^+$)

Beispiel 11a-11r

Die Verbindungen Ia-Ir wurden in analoger Weise, wie in Versuchsvorschrift 11 beschrieben, hergestellt (vgl. Tabelle 13).

Tabelle 13

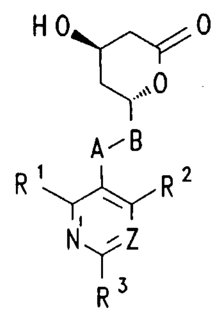

A – B = –CH=CH–

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: $\delta$/ppm =<br>MS : m/e = |
|---|---|---|---|---|---|---|---|---|
| a | Ia | CH | $iC_3H_7$ | 4-$FC_6H_4$ | $C_6H_5$ | 63 | -; 0.22[c]<br>Öl;<br>-; +24°($CH_3$-OH) | E: 1.4 (d,J=7Hz,6H), 1.5 (brs,1H), 1.6 (mc, 1H), 1.8-1.9 (m,1H), 2.6-2.8 (m,2H), 3.4 (h,J=7Hz,1H), 4.3 (mc,1H), 5.2 (mc,1H), 5.4 (mc,1H), 6.7 (d,J=16Hz,1H), 7.1 (mc,2H), 7.3-7.5 (m,6H), 8.1 (mc, 2H).<br>431 ($M^+$) |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: $\delta$/ppm =<br>MS : m/e = |
|---|---|---|---|---|---|---|---|---|
| b | Ib | CH | 4-$FC_6H_4$ | $iC_3H_7$ | $C_6H_5$ | 70 | -; 0.15[c]<br>-; Öl<br>-; - | E: 1.5 (d,J=7Hz,6H), 1.5-1.8 (m,3H), 2.5-2.7 (m,2H), 3.4 (h,J=7Hz,1H), 4.3 (mc,1H), 5.2 (mc,1H), 5.4 (mc,1H), 6.7 (d,J=16Hz,1H), 7.0-8.2 (m,10H).<br>431 ($M^+$) |
| c | Ic | CH | $tC_4H_9$ | 4-$FC_6H_4$ | $C_6H_5$ | 64 | -; 0.27[c]<br>-; Öl<br>-; - | E: 1.5 (s,9H), 1.5-2.8 (m,5H), 4.2 (mc,1H), 5.2 (mc,1H), 5.5 (mc,1H), 6.7 (d,J=16Hz,1H), 7.0-7.6(m,8H), 8.0-8.2 (m,2H).<br>445 ($M^+$) |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: $\delta$/ppm =<br>MS : m/e = |
|---|---|---|---|---|---|---|---|---|
| d | Id | CH | $iC_3H_7$ | $4\text{-}CH_3OC_6H_4$ | $C_6H_5$ | 77 | -; 0.26[c]<br>-; Öl<br>-; - | E: 1.5 (d,J=7Hz,6H), 1.4-1.8 (m,3H), 2.5-2.8 (m,2H), 3.6 (h,J=7Hz,3H), 3.9 (s, 3H), 4.3 (mc,1H), 5.2 (mc,1H), 5.5 (mc,1H), 6.7 (d,J=16Hz,1H), 7.0-7.6 (m,8H), 8.1-8.2 (m,2H).<br>443 ($M^+$) |
| e | Ie | CH | $iC_3H_7$ | $4\text{-}FC_6H_4$ | $2,5\text{-}(CH_3)_2\text{-}C_6H_3$ | 71 | -; 0.30[c]<br>-; Öl<br>-; - | E: 1.3 (d,J=7Hz,6H), 1.5-1.8 (m,3H), 2.3 (s,3H), 2.4 (s, 3H), 2.6-2.8 (m,2H), 3.5 (h,J=7Hz,1H), 4.3 (mc,1H), 5.2 (mc,1H), 5.5 (mc,1H), 6.6 (d,J=16Hz,1H), 6.9-7.5 (m,8H).<br>459 ($M^+$) |

| Bsp. | Verb. | Z | $R^1$ | $R^2$ | $R^3$ | Ausb. % | $R_f$-Wert (Z;E)<br>Fp °C (Z;E)<br>$[\alpha]_D^{25}$(Z;E) | $^1$H-NMR: $\delta$/ppm =<br>MS : m/e = |
|---|---|---|---|---|---|---|---|---|
| f | If | CH | $iC_3H_7$ | 4-F-$C_6H_4$ | 4-$FC_6H_4$ | 65 | -; 0.20[c]<br>-; Öl | E: 1.4 (d,J=7Hz,6H), 1.5-1.9 (m, 3H), 2.6-2.8 (m,2H), 3.5 (h,J=7Hz, 1H), 4.3 (mc,1H), 5.2 (mc,1H), 5.5 (mc,1H), 6.7 (d,J=16Hz,1H), 7.0-8.1 (m,9H).<br>449 ($M^+$) |
| g | Ig | N | $iC_3H_7$ | 4-$FC_6H_4$ | $C_6H_5$ | 93 | 0.10[d]<br>-; 164-166<br>-; +14 ($CH_3$-OH) | 1.5 (d,J=7Hz,6H), 1.6 (s,1H), 1.7-2.0 (m, 2H), 2.6-2.9 (m,2H), 3.4 (h, J=7Hz,1H), 4.3 (mc,1H), 5.3 (mc, 1H), 5.6 (mc, 1H), 6.8 (dd,J=16Hz, 1H), 7.2-7.7 (m,7H), 8.6 (mc,2H). |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: $\delta$/ppm =<br>MS : m/e = |
|---|---|---|---|---|---|---|---|---|
| h | Ih | CH | $iC_3H_7$ | 4-$FC_6H_4$ | $iC_3H_7$ | 71 | -; -<br>-; 137-140<br>-; - | 1.2-1.3 (m,12H), 1.5-1.7 (m,2H), 2.5-2.8 (m,2H), 3.0 (h,J=7Hz,3H), 3.3 (h,J=7Hz,1H), 4.2 (mc,1H), 5.1 (mc,1H), 5.3 (mc,1H), 6.6 (dd, J=16Hz,1H), 7.1 (mc,2H), 7.3 (mc, 2H).<br>398 ($M^+$+H) |
| i | Ii | CH | $iC_3H_7$ | 4-$FC_6H_4$ | $tC_4H_9$ | 78 | -; 0.35[c]<br>-; 158-160<br>-; + 26.4° ($CH_3OH$)<br>-; Öl | 1.3 (mc,6H), 1.4 (s,9H), 1.5-1.7 (m,3H), 1.8 (mc,1H), 2.6 (mc,1H), 2.7 (mc,1H), 3.3 (h,J=7Hz,3H), 4.3 (mc,1H), 5.2 (mc,1H), 5.4 (dd,J=16Hz,6Hz,1H), 6.6 (dd,J=16Hz,1Hz,1H), 7.0 (s,1H), 7.1 (mc, 2H), 7.3 (mc,2H)<br>412 ($M^+$+H) |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: δ/ppm =<br>MS : m/e = |
|---|---|---|---|---|---|---|---|---|
| j | Ij | CH | $iC_3H_7$ | $4\text{-}FC_6H_4$ | $cC_6H_{11}$ | 65 | -; 0.30[c]<br>-; 135-138<br>-; - | 1.2 (mc,6H), 1.3-2.0 (m,14H), 2.0 2.6 (mc,1H), 2.7 (mc,1H), 3.3 (h,J=7Hz,1H), 4.2 (mc,1H), 5.1 (mc,1H), 5.3 (dd,J=16Hz,5Hz,1H), 6.6 (dd,J=16Hz,1Hz,1H), 6.8 (s, 1H), 7.1 (mc,2H), 7.2 (mc,2H). |
| k | Ik | CH | $cC_6H_{11}$ | $4\text{-}FC_6H_4$ | $C_6H_5$ | 45 | -; 0.32[f]<br>-; 196-198<br>-; Öl | 1.3-2.0 (m,13H), 2.6 (mc,1H), 2.7 (mc,1H), 3.0 (mc,1H), 4.3 (mc,1H), 5.2 (mc,1H), 5.4 (mc,1H), 6.7 (dd,J=16Hz,1Hz,1H), 7.0 (mc,2H), 7.3-7.5 (m,6H), 8.1 (mc,2H).<br>472 ($M^+$+H) |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: δ/ppm =<br>MS : m/e = |
|---|---|---|---|---|---|---|---|---|
| I | II | N | $iC_3H_7$ | 4-$FC_5H_4$ | $iC_3H_7$ | 71 | -; 0.16[e]<br>-; Öl | 1.3 (d,6H), 1.5 (d,6H), 1.5-1.9 (m,2H), 2.6-2.8 (m,2H), 3.2-3.32 (h,1H), 4.3 (m,1H), 5.25 (m,1H), 5.5 (dd,1H), 6.7 (dd,J=16Hz,1Hz, 1H), 7.1-7.5 (m,4H)<br>399 ($M^+$+H) |
| m | Im | N | $iC_3H_7$ | 4-$FC_6H_4$ | 4-$FC_6H_4$ | 48 | -; 0.24[e]<br>-; 138-140 | 1.48 (d,6H), 1.5-2.0 (m,2H), 2.6-2.8 (m,2H), 3.40 (h,1H), 4.35 (m,1H), 5.25 (m,1H), 5.5 (dd,J=16Hz,1H), 6.78 (dd,J=16Hz, 1Hz,1H), 7.15-7.6 (m,4H). |

| Bsp. | Verb. | Z | $R^1$ | $R^2$ | $R^3$ | Ausb. % | $R_f$-Wert (Z;E)<br>Fp°C (Z;E)<br>$[\alpha]_D^{25}$(Z;E) | $^1$H-NMR: $\delta$/ppm =<br>MS : m/e = |
|---|---|---|---|---|---|---|---|---|
| n | In | CH | $iC_3H_7$ | $4\text{-}HOC_6H_4$ | $C_6H_5$ | - | 0.45[d]<br>180-182 | 1.4 (d,J=7Hz,6H), 1.6-1.9 (m,2H), 2.6 (mc,1H), 2.7 (mc,1H), 3.4 (h, J=7Hz,1H), 4.2 (mc,1H), 5.2 (mc, 1H), 5.4 (dd,J=16Hz,6H,1H), 6.7 (dd,J= 16Hz,1Hz,1H), 6.9 (mc,2H), 7.2 (mc,2H), 7.4-7.5 (m,3H), 7.5 (s,1H), 8.1 (mc,2H).<br>430 ($M^+$+H) |
| o | Io | CH | $iC_3H_7$ | $4\text{-}FC_6H_4$ | $4\text{-}HOC_6H_4$ | - | 0.22[e]<br>185-187<br>(Zers.) | 1.3 (mc,6H), 1.5 (brs,2H), 1.6 (mc, 1H), 1.8 (mc,1H), 2.6 (mc,1H), 2.7 (mc,1H), 3.5 (h,J=7Hz,1H), 4.3 (mc,1H), 5.2 (mc, 1H), 5.4 (dd,J= 16Hz,6Hz,1H), 6.7 (dd,J=16Hz, 1Hz,1H), 6.9 (mc,2H), 7.1 (mc,2H), 7.3 (mc,2H), 7.4 (s,1H), 8.0 (mc, 2H).<br>448 ($M^+$ +H) |

| Bsp. | Verb. | Z | $R^1$ | $R^2$ | $R^3$ | Ausb. % | $R_f$-Wert (Z;E)<br>Fp °C (Z;E)<br>$[\alpha]_D^{25}$(Z;E) | $^1$H-NMR: $\delta$/ppm =<br>MS : m/e = |
|---|---|---|---|---|---|---|---|---|
| p | Ip | CH | $cC_3H_5$ | $4\text{-}FC_6H_4$ | $C_6H_5$ | - | -; 0.20[c]<br><br>-; Öl | 1.0 (mc,2H), 1.3 (mc,2H), 1.6-1.9 (m,2H), 2.6 (mc,1H), 2.7 (mc,1H), 4.2 (mc,1H), 5.2 (mc,1H), 5.4 (dd, J = 16Hz,6Hz,1H), 6.7 (dd,J = 16Hz, 1Hz,1H), 7.1 (mc,2H), 7.3-7.5 (m,6H), 8.0 (mc, 2H).<br>430 ($M^+$ + H) |

[a]Ethylacetat/Methanol 10:1 [b]Cyclohexan/Ethylacetat 1:4 [c]Cyclohexan/Ethylacetat 2:1 [d] Ethylacetat [e]Cyclohexan/Ethylacetat 1:1 [f]Cyclohexan/Ethylacetat 4:1

Beispiel 12

Hydrierung von Verbindungen der allgemeinen Formel I mit A-B = -CH = CH- zu Verbindungen der Formel I mit A-B = $-CH_2\text{-}CH_2-$

Versuchvorschrift 12

(4R,6S)-(6-(2-(2,6-Dimethyl-4-(4-fluorphenyl)pyrimidin-3-yl)ethyl-4-hydroxy-3,4,5,6-tetrahydro-2H-pyran-2-on (Ix) ($R^1$ = $CH_3$, $R^2$ = 4-$FC_6H_4$, $R^3$ = $CH_3$, Z = N, A-B = -CH2-CH2-)
4.00 g (11.7 mmol) der Verbindung E-I ($R^1$ = $CH_3$, $R^2$ = 4-$FC_6H_4$, $R^3$ = $CH_3$, Z = N, A-B = -CH = CH-) wurden in einem Gemisch aus 50 ml Methanol und 50 ml Ethylacetat gelöst und nach Zusatz von 50 mg Palladium auf Kohle/10 %, sowie 50 µl Triethylamin, in einer Wasserstoffatmosphäre geschüttelt, bis kein $H_2$ mehr aufgenommen wurde. Die Reaktionslösung wurde über Kieselgur filtriert und eingedampft. Zurück blieb I ($R^1$ - $CH_3$, $R^2$ 4-$FC_6H_4$, $R^3$ = $CH_3$, Z = N, A-B = -$CH_2$ = $CH_2$-) in Form weißer Kristalle.
Ausbeute: 3.93 g (98 %)
Fp: 170-172°C
$[\alpha]_D^{25}$ ($CH_3OH$): + 13°

$^1$H-NMR: δ/ppm = 1.5-1.9 (m,2H), 1.9 (brs,1H), 2.6 (s,3H), 2.7 (s,3H), 2.6-3.0 (m,2H), 4.3 (m,1H), 4.5-4.6 (m,1H), 7.1-7.2 (m,2H), 7.4-7.5 (m,2H).

Beispiel 12

Unter den in Versuchsvorschrift 12 angegebenen Bedingungen wurden 1.0 g der Verbindung E-I ($R^1$ = $iC_3H_7$, $R^2$ = 4-$FC_6H_4$, $R^3$ = $C_6H_5$, Z = CH, A-B = -CH = CH-) (Beispiel 11) zum Hydrierungsprodukt I ($R^1$ = $iC_3H_7$, $R^2$ = 4-$FC_6H_4$, $R^3$=$C_6H_5$, Z = CH, A-B = -$CH_2$-$CH_2$) umgesetzt.
Ausbeute: 0.91 g (91 %)
Fp: Öl
$[\alpha]_D^{25}$ ($CH_3OH$): + 26°

$^1$H-NMR: δ/ppm = 1.3-1.8 (m,11H), 2.3-2.8 (m, 7H), 3.4 (h,J = 7Hz,1H), 4.2 (mc,1H), 4.5 (mc,1H), 7.1 (mc,2H), 7.3-7.5 (m,6H), 8.1 (mc,2H).
MS: m/e = 433 ($M^+$)

In analoger Weise, wie in Beispiel 12 beschrieben, können die Verbindungen der allgemeinen Formel I mit A-B = CH = CH- zu Verbindungen der allgemeinen Formel I mit A-B = $CH_2$-$CH_2$- hydriert werden.

Beispiel 13

Herstellung der Salze der freien Dihydroxysäuren der allgemeinen Formel II

Versuchsvorschrift 13 ($R^1$ = $CH_3$, $R^2$ = 4-$FC_6H_4$, $R^3$ = $CH_3$, $R^4$ = K, Z = CH, A-B = (E)-CH = CH-)
(E)- und (Z)-(3R,5S)-3,5-Dihydroxy-7-(2,6-Dimethyl-4-(4-fluorphenyl)pyridin-3-yl)-hept-6-ensäure Kaliumsalze E-II und Z-II (als 30:70 Gemisch von Z- und E-Isomer)
0.10 g (0,29 mmol) der Verbindung I wurden in 5 ml Ethanol gelöst. Zu dieser Lösung wurden bei Raumtemperatur 2.9 ml (0.29 mmol) einer 0.1 molaren Lösung von Kaliumhydroxid in Ethanol gegeben. Das Fortschreiten der Umsetzung wurde dünnschichtchromatographisch (Laufmittel Ethylacetat/Methanol 10:1) verfolgt. Nach 3 h war kein Edukt mehr vorhanden. Die Reaktionslösung wurde im Vakuum eingeengt. Zurück blieb das Kaliumsalz II in Form weißer Kristalle.
Ausbeute: 0.11 g (96 %) (30:70-Gemisch aus Z-IIa und E-IIa)
Die Isomeren wurden anschließend durch Mitteldruckflüssigkeitschromatographie getrennt.

Z-II: $R_f$-Wert (Ethylacetat/Methanol 2:1): 0.23
IR: 1605/1575 $cm^{-1}$ (C = O-Bande)
E-II: $R_f$-Wert (Ethylacetat/Methanol 2:1): 0.19
IR: 1610/1580 $cm^{-1}$ (C = O-Bande)

Beispiel 13a - 13q

Die Verbindungen IIa-IIq wurden in analoger Weise, wie in Versuchsvorschrift 13 beschrieben, hergestellt (vgl. Tabelle 14).

Tabelle 14

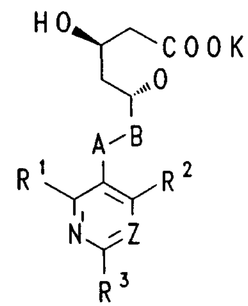

A − B = (E)−CH=CH, (Z)−CH=CH

| Bsp. | Verb. | Z | $R^1$ | $R^2$ | $R^3$ | Ausb. % | $R_f$-Wert (Z;E) |
|---|---|---|---|---|---|---|---|
| a | IIa | CH | $iC_3H_7$ | $4\text{-}FC_6H_4$ | $C_6H_5$ | 99 | -; 0.44[a] |
| b | IIb | CH | $4\text{-}FC_6H_4$ | $iC_3H_7$ | $C_6H_5$ | 91 | -; 0.38[a] |
| c | IIc | CH | $tC_4H_9$ | $4\text{-}FC_6H_4$ | $C_6H_5$ | 97 | -; 0.50[a] |
| d | IId | CH | $iC_3H_7$ | $4\text{-}CH_3OC_6H_4$ | $C_6H_5$ | 96 | -; 0.47[a] |

| Bsp. | Verb. | Z | $R^1$ | $R^2$ | $R^3$ | Ausb. % | $R_f$-Wert (Z;E) |
|---|---|---|---|---|---|---|---|
| e | IIe | CH | $iC_3H_7$ | $4\text{-}FC_6H_4$ | $2,5\text{-}(CH_3)_2\text{-}C_6H_3$ | 100 | -; 0.56[a] |
| f | IIf | CH | $iC_3H_7$ | $4\text{-}FC_6H_4$ | $4\text{-}FC_6H_4$ | 94 | -; 0.40[a] |
| g | IIg | N | $iC_3H_7$ | $4\text{-}FC_6H_4$ | $C_6H_5$ | 97 | 0.35[a] |
| h | IIh | CH | $iC_3H_7$ | $4\text{-}FC_6H_4$ | $iC_3H_7$ | 95 | -; 0.49[a] |
| i | IIi | CH | $iC_3H_7$ | $4\text{-}FC_6H_4$ | $tC_4H_9$ | 98 | -; 0.53[a] |
| j | IIj | CH | $iC_3H_7$ | $4\text{-}FC_6H_4$ | $cC_6H_{11}$ | 96 | -; 0.50[a] |
| k | IIk | CH | $cC_6H_{11}$ | $4\text{-}FC_6H_4$ | $C_6H_5$ | 100 | -; 0.64[a] |
| l | IIl | N | $iC_3H_7$ | $4\text{-}FC_5H_4$ | $iC_3H_7$ | 100 | -; 0.26[b] |

| Bsp. | Verb. | Z | $R^1$ | $R^2$ | $R^3$ | Ausb. % | $R_f$-Wert (Z;E) |
|---|---|---|---|---|---|---|---|
| m | IIm | N | $iC_3H_7$ | 4-$FC_6H_4$ | 4-$FC_6H_4$ | 100 | -; 0.11[b] |
| n | IIn A-B = $CH_2$-$CH_2$ | CH | $iC_3H_7$ | 4-$FC_6H_5$ | $C_6H_5$ | 98 | -; 0.29[a] |
| o | IIo | CH | $iC_3H_7$ | 4-$HOC_6H_4$ | $C_6H_5$ | 69 | -; 0.30[a] |
| p | IIp | CH | $iC_3H_7$ | 4-$FC_6H_4$ | 4-$HOC_6H_4$ | 83 | -; 0.18[a] |
| q | IIq | CH | $cC_3H_7$ | 4-$FC_6H_4$ | $C_6H_5$ | 96 | -; 0.40[a] |

[a]Ethylacetat/Methanol 2:1 [b] Dichlormethan/Methanol 9:1

Beispiel 14

Allgemeine Vorschrift zur Darstellung von Carbonsäureestern der freien Dihydroxysäuren der allgemeinen Formel II

Versuchsvorschrift 14 ($R^1$ = $CH_3$, $R^2$ = 4-$FC_6H_4$, $R^3$ = $CH_3$, $R^4$ = $CH_3$, Z = CH, A-B=(E)-CH = CH)
E-(3R,5S)-3,5-Dihydroxy-7-(2,6-Dimethyl)-4-(4-fluorphenyl)pyridin-3-yl)-hept-6-ensäure methylester E-IIad
0.40 g (1.17 mmol) der Verbindung E-I ($R^1$ = $CH_3$, $R^2$ = 4-$FC_6H_4$, $R^3$ = $CH_3$, A-B = -CH = CH-) (Beispiel 11) wurden in 10 ml Methanol gelöst und bei Raumtemperatur mit 1.3 ml (0.13 mmol) einer 0.1 molaren Lösung von Natriummethanol in Methanol versetzt. Nach einstündigem Rühren - der Umsatz der Reaktion wurde dünnschichtchromatographisch (Laufmittel/Ethylacetat) verfolgt - wurde das Lösungsmittel im Vakuum entfernt. Der Rückstand wurde in Wasser aufgenommen, mit Essigsäure neutralisiert und ausgeethert. Die organischen Extrakte wurden über Magnesiumsulfat getrocknet und eingedampft. Zurück blieben 0.39 g (94 %) der Titelverbindung E-IIad.

$^1$H-NMR (Auszug): δ/ppm = 3.6 (s,3H)

In analoger Weise, wie in Versuchsvorschrift 14 beschrieben, können die Methylester der freien Dihydroxysäuren der allgemeinen Formel II hergestellt werden. Ersetzt man Methanol durch andere Alkohole ($R^4$OH) sind auch entsprechende andere Ester II ($R^4$ = Ethyl, iso Propyl, Benzyl etc.) leicht darstellbar.

## Patentansprüche

**Patentansprüche für folgende Vertragsstaaten : AT, BE, CH, DE, FR, GB, IT, LI, LU, NL, SE**

1. 3-Desmethyl-mevalonsäurederivate der Formel I (δ-Lacton) bzw. II (entsprechendes Dihydroxycarbonsäurederivat)

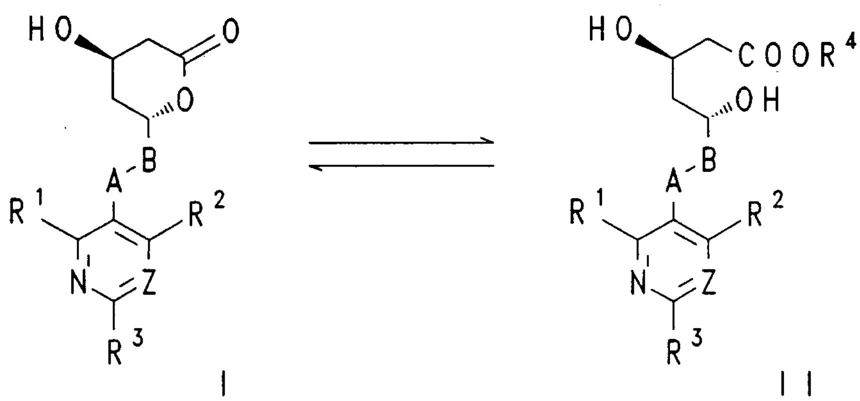

I    II

worin bedeuten

| | |
|---|---|
| A-B | einen Rest der Formel -CH = CH- oder $-CH_2-CH_2-$ |
| Z | einen Rest der Formel -CH oder ein Stickstoffatom |
| $R^1$, $R^2$, $R^3$ | unabhängig voneinander einen verzweigten Alkylrest mit bis zu 4 C-Atomen, einen Cycloalkylrest mit 3-6 C-Atomen oder einen Phenylrest, der gegebenenfalls 1 bis 2 gleiche oder verschiedene Substituenten ausgewählt aus der Gruppe Fluor, Hydroxy und $C_1$-$C_4$-Alkyl trägt, |
| $R^4$ | Wasserstoff, Methyl, Ethyl, Isopropyl, Isobutyl, Benzyl, Natrium, Kalium, Ammonium ($NH_4$) oder Methyltris(hydroxymethyl)ammonium. |

2. Verbindungen gemäß Anspruch 1, dadurch gekennzeichnet, daß in Formel I bzw. Formel II

| | |
|---|---|
| $R^1$ | Isopropyl, sek.-Butyl, tert.-Butyl, Cyclopropyl, Cyclohexyl, Phenyl, 4-Fluorphenyl, 4-Hydroxyphenyl, 4-Fluor-3-methylphenyl, 3,5-Dimethylphenyl, Cyclohexylmethyl. |

$R^2$ Isopropyl, sek.-Butyl, tert.-Butyl, Cyclopropyl, Cyclohexyl, Phenyl, 4-Fluorphenyl, 4-Hydroxyphenyl, 4-Fluor-3-methylphenyl, 3,5-Dimethylphenyl, Cyclohexylmethyl.

$R^3$ Isopropyl, tert.-Butyl, Cyclohexyl, Phenyl, 4-Fluorphenyl, 4-Hydroxyphenyl, 2,5-Dimethylphenyl, 3,5-Dimethylphenyl.

$R^4$ Wasserstoff, Methyl, Ethyl, Natrium, Kalium darstellt.

**3.** Verbindungen gemäß Anspruch 1, dadurch gekennzeichnet, daß in Formel I bzw. Formel II

Z die CH-Gruppe oder N
$R^1$ Isopropyl, Cyclopropyl
$R^2$ 4-Fluorphenyl, 4-Hydroxyphenyl
$R^3$ Isopropyl, tert.-Butyl, Cyclohexyl, Phenyl, 4-Fluorphenyl oder 4-Hydroxyphenyl und
$R^4$ Natrium oder Kalium darstellen.

**4.** Verfahren zur Herstellung von Verbindungen der Formel I und II dadurch gekennzeichnet, daß man

a) die Phosphoniumsalze der Formel III

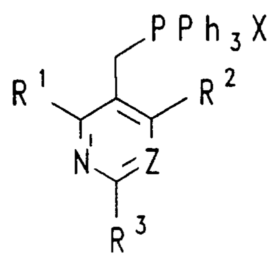

III

worin $R^1$, $R^2$, $R^3$ und Z die zur Formel I angegebene Bedeutung haben und X = Cl, Br, I ist, mit dem chiralen Aldehyd der Formel IV umsetzt,

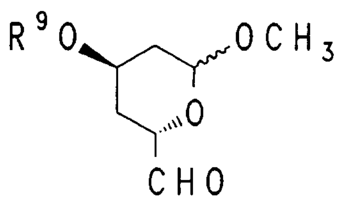

IV

worin $R^9$ eine gegen Basen und schwache Säuren stabile Schutzgruppe bedeutet, zu einer Verbindung der

Formel V

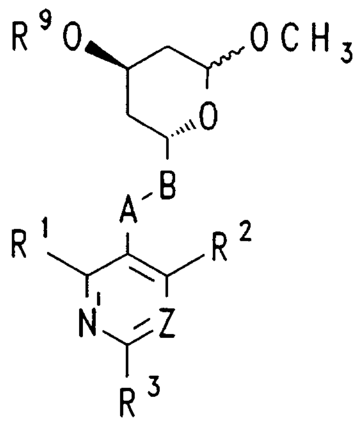

V

worin $R^1$, $R^2$, $R^3$ und Z die zur Formel I, $R^9$ die zur Formel IV gegebene Bedeutung haben (und A-B die (-CH = CH-)-Gruppe darstellt),

b) in einer Verbindung der allgemeinen Formel V die Methylacetalfunktion sauer hydrolysiert zu einem Lactol der Formel VI,

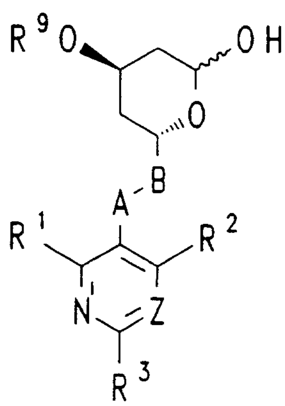

V I

worin $R^1$, $R^2$, $R^3$ und Z die zur Formel I, $R^9$ die zur Formel IV angegebene Bedeutung haben und A-B die (-CH = CH-)-Gruppe darstellt,

c) die Verbindung der allgemeinen Formel VI oxidiert zu einem Lacton der Formel VII,

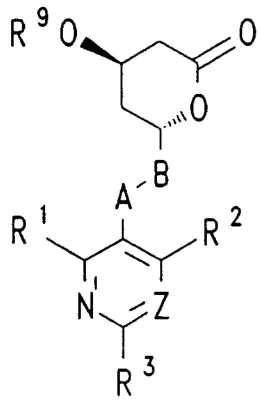

VII

worin $R^1$, $R^2$, $R^3$ und Z die zur Formel I, $R^9$ die zur Formel IV angegebene Bedeutung haben und A-B die (-CH = CH-)-Gruppe darstellt und

d) in einer Verbindung der allgemeinen Formel VII die Schutzgruppe $R^9$ abspaltet zu einer Verbindung der Formel I, worin $R^1$, $R^2$, $R^3$ und Z die zur Formel I angegebenen Bedeutungen haben und A-B die (-CH = CH-)-Gruppe darstellt, und

e) gegebenenfalls eine enthaltene Verbindung der allgemeinen Formel I, bei der A-B eine (-CH = CH)-Gruppe darstellt, hydriert zu einer Verbindung der allgemeinen Formel I, in der A-B eine (-$CH_2$-$CH_2$-)-Gruppe darstellt, wobei die Hydrierung auch bei den Verbindungen der Formel V, VI oder VII erfolgen kann zu entsprechenden Verbindungen, worin A-B die (-$CH_2$-$CH_2$-)-Gruppe darstellt, und

f) gegebenenfalls ein Hydroxylacton der Formel I in die entsprechende Dihydroxysäure II bzw. deren Salze überführt oder gegebenenfalls aus dem Hydroxylacton I oder der freien Hydroxysäure II die entsprechenden Ester darstellt.

**5.** Pharmazeutisches Präparat, dadurch gekennzeichnet, daß es eine Verbindung gemäß Anspruch 1 enthält.

**6.** Verwendung von Verbindungen gemäß Anspruch 1 zur Prophylaxe und Therapie der Arteriosklerose und Hypercholesterinämie.

**7.** Verbindungen der Formel V

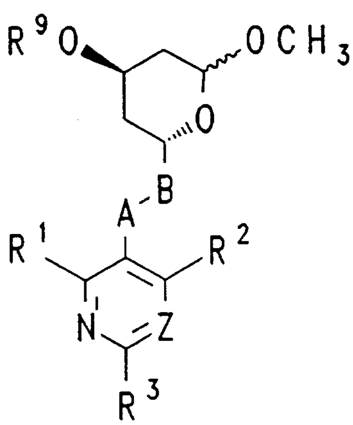

V

worin $R^1$, $R^2$, $R^3$, A-B und Z die zur Formel I, $R^9$ die zur Formel IV angegebene Bedeutung haben.

**8.** Verbindungen der Formel VI

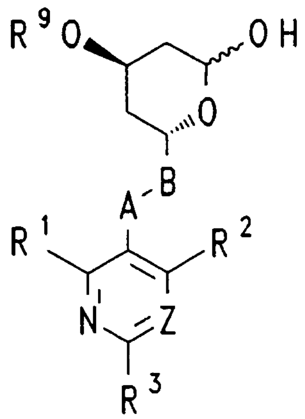

VI

worin $R^1$, $R^2$, $R^3$, A-B und Z die zur Formel I, $R^9$ die zur Formel IV angegebene Bedeutung haben.

**9.** Verbindungen der Formel VII

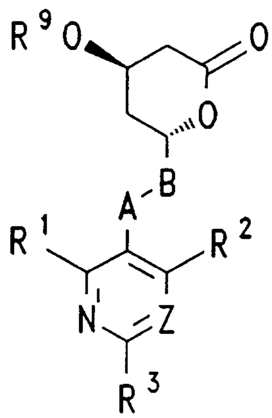

VII

worin $R^1$, $R^2$, $R^3$, A-B und Z die zur Formel I, $R^9$ die zur Formel IV angegebene Bedeutung haben.

**Patentansprüche für folgende Vertragsstaaten : ES, GR**

**1.** Verfahren zur Herstellung von 3-Desmethyl-mevalonsäurederivaten der Formel I (δ-Lacton) bzw. II (entsprechendes Dihydroxycarbonsäurederivat)

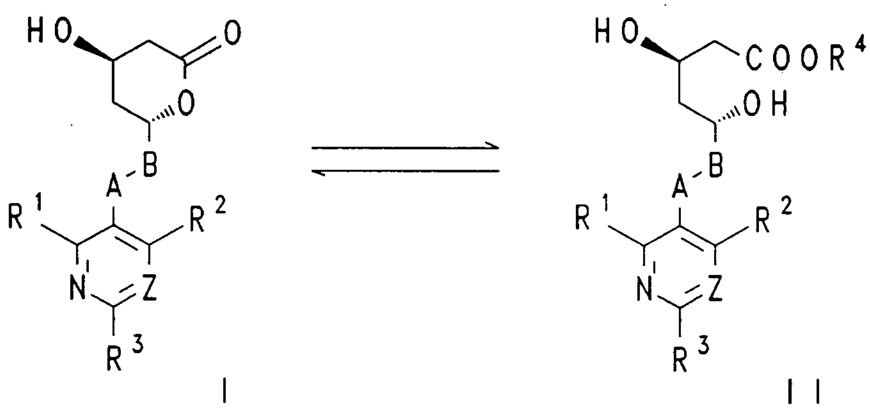

I II

worin bedeuten

| | |
|---|---|
| A-B | einen Rest der Formel -CH = CH- oder $-CH_2-CH_2-$ |
| Z | einen Rest der Formel -CH oder ein Stickstoffatom |
| $R^1$, $R^2$, $R^3$ | unabhängig voneinander einen verzweigten Alkylrest mit bis zu 4 C-Atomen, einen Cycloalkylrest mit 3-6 C-Atomen oder einen Phenylrest, der gegebenenfalls 1 bis 2 gleich oder verschiedene Substituenten ausgewählt aus der Gruppe Fluor, Hydroxy und $C_1$-$C_4$-Alkyl trägt, |
| $R^4$ | Wasserstoff, Methyl, Ethyl, Isopropyl, Isobutyl, Benzyl, Natrium, Kalium, Ammonium ($NH_4$) oder Methyltris(hydroxymethyl)ammonium, dadurch gekennzeichnet, daß man |

a) die Phosphoniumsalze der Formel III

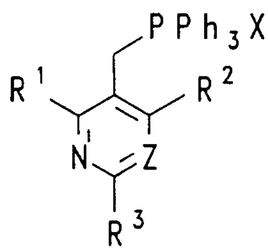

III

worin $R^1$, $R^2$, $R^3$ und Z die zur Formel I angegebene Bedeutung haben und X = Cl, Br, I ist, mit dem chiralen

Aldehyd der Formel IV umsetzt,

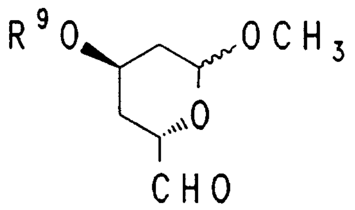

I V

worin $R^9$ eine gegen Basen und schwache Säuren stabile Schutzgruppe bedeutet, zu einer Verbindung der Formel V

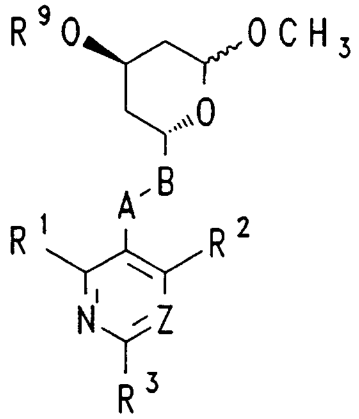

V

worin $R^1$, $R^2$, $R^3$ und Z die zur Formel I, $R^9$ die zur Formel IV angegebene Bedeutung haben (und A-B die (-CH = CH-)-Gruppe darstellt),

b) in einer Verbindung der allgemeinen Formel V die Methylacetalfunktion sauer hydrolysiert zu einem Lactol der Formel VI,

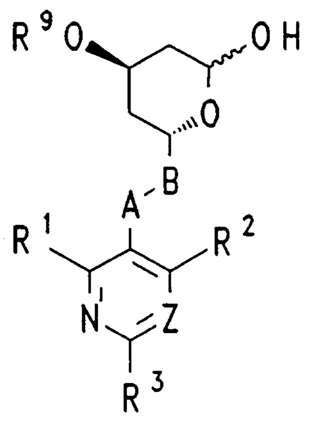

V I

worin $R^1$, $R^2$, $R^3$ und Z die zur Formel I, $R^9$ die zur Formel IV angegebene Bedeutung haben und (A-B die (-CH = CH-)-Gruppe darstellt),

c) die Verbindung der allgemeinen Formel VI oxidiert zu einem Lacton der Formel VII,

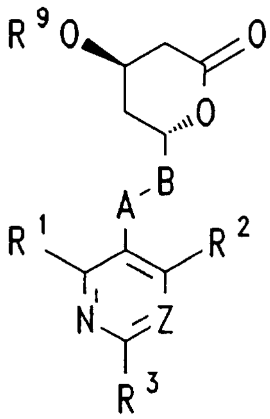

VII

worin $R^1$, $R^2$, $R^3$ und Z die zur Formel I, $R^9$ die zur Formel IV angegebene Bedeutung haben und A-B die (-CH = CH-)-Gruppe darstellt und

d) in einer Verbindung der allgemeinen Formel VII die Schutzgruppe $R^9$ abspaltet zu einer Verbindung der Formel I, worin $R^1$, $R^2$, $R^3$ und Z die zur Formel I angegebenen Bedeutungen haben und A-B die (-CH = CH-)-Gruppe darstellt, und e) gegebenenfalls eine enthaltene Verbindung der allgemeinen Formel I, bei der A-B eine (-CH = CH)-Gruppe darstellt, hydriert zu einer Verbindung der allgemeinen Formel I, in der A-B eine (-$CH_2$-$CH_2$-)-Gruppe darstellt, wobei die Hydrierung auch bei den Verbindungen der Formel V, VI oder VII erfolgen kann zu entsprechenden Verbindungen, worin A-B die (-$CH_2$-$CH_2$-)-Gruppe darstellt, und

f) gegebenenfalls ein Hydroxylacton der Formel I in die entsprechende Dihydroxysäure II bzw. deren Salze überführt oder gegebenenfalls aus dem Hydroxylacton I oder der freien Hydroxysäure II die entsprechenden Ester darstellt.

**2.** Verfahren gemäß Anspruch 1, dadurch gekennzeichnet, daß man eine Verbindung der Formel I bzw. Formel II, worin

$R^1$ und $R^2$ unabhängig voneinander Isopropyl, sek.-Butyl, tert.-Butyl, Cyclopropyl, Cyclohexyl, Phenyl, 4-Fluorphenyl, 4-Hydroxyphenyl, 4-Fluor-3-methylphenyl, 3,5-Dimethylphenyl, Cyclohexylmethyl bedeuten,

$R^3$ Isopropyl, tert.-Butyl, Cyclohexyl, Phenyl, 4-Fluorphenyl, 4-Hydroxyphenyl, 2,5-Dimethylphenyl, 3,5-Dimethylphenyl darstellt,

$R^4$ Wasserstoff, Methyl, Ethyl, Natrium, Kalium ist, herstellt.

**3.** Verfahren gemäß Anspruch 1, dadurch gekennzeichnet, daß man eine Verbindung der Formel I bzw. Formel II, worin

$R^1$ Isopropyl, Cyclopropyl
$R^2$ 4-Fluorphenyl, 4-Hydroxyphenyl
$R^3$ Isopropyl, tert.-Butyl, Cyclohexyl, Phenyl, 4-Fluorphenyl oder 4-Hydroxyphenyl und
$R^4$ Natrium oder Kalium darstellt, herstellt.

**4.** Verfahren gemäß Anspruch 1, dadurch gekennzeichnet, daß man Verbindungen der Formel V

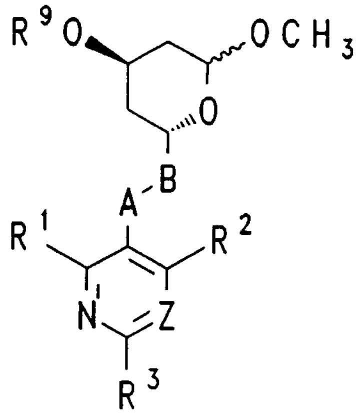

V

worin $R^1$, $R^2$, $R^3$, A-B und Z die zur Formel I, $R^9$ die zur Formel IV angegebene Bedeutung haben, isoliert.

**5.** Verfahren gemäß Anspruch 1, dadurch gekennzeichnet, daß man Verbindungen der Formel VI

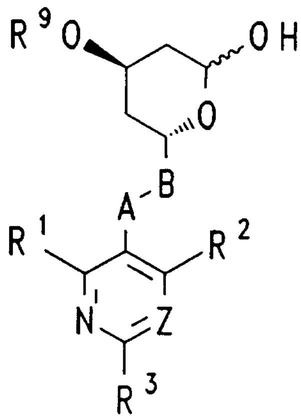

VI

worin $R^1$, $R^2$, $R^3$, A-B und Z die zur Formel I, $R^9$ die zur Formel IV angegebene Bedeutung haben, isoliert.

**6.** Verfahren gemäß Anspruch 1, dadurch gekennzeichnet, daß man Verbindungen der Formel VII

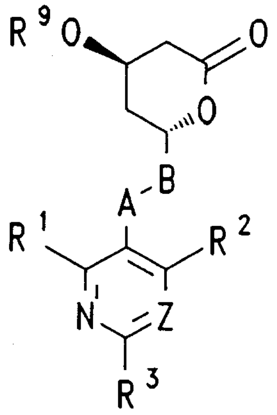

VII

worin $R^1$, $R^2$, $R^3$, A-B und Z die zur Formel I, $R^9$ die zur Formel IV angegebene Bedeutung haben, isoliert.

7. Verfahren gemäß Anspruch 1, dadurch gekennzeichnet, daß man eine erhaltene Verbindung der Formel I oder II in einem weiteren Verfahrensschritt in eine geeignete Darreichungsform überführt.

## Claims

### Claims for the following Contracting States : AT, BE, CH, DE, FR, GB, IT, LI, LU, NL, SE

1. A 3-demethylmevalonic acid derivative of the formula I (δ-lactone) or II (corresponding dihydroxy carboxylic acid derivative)

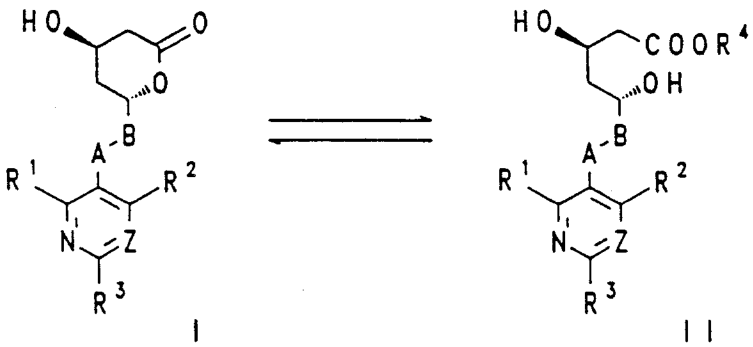

I II

in which

A-B denotes a radical of the formula -CH=CH- or $-CH_2-CH_2-$,

Z denotes a radical of the formula -CH or a nitrogen atom,

$R^1$, $R^2$ and $R^3$, independently of one another, denote a branched alkyl radical having up to 4 carbon atoms, a cycloalkyl radical having 3-6 carbon atoms or a phenyl radical which optionally carries 1 to 2 identical or different substituents selected from the group consisting of fluorine, hydroxyl and $C_1$-$C_4$-alkyl,
$R^4$ is hydrogen, methyl, ethyl, isopropyl, isobutyl, benzyl, sodium, potassium, ammonium ($NH_4$) or methyl-tris(hydroxymethyl)ammonium.

2. A compound as claimed in claim 1, wherein, in formula I or formula II,
$R^1$ represents isopropyl, sec.-butyl, tert.-butyl, cyclopropyl, cyclohexyl, phenyl, 4-fluorophenyl, 4-hydroxyphenyl, 4-fluoro-3-methylphenyl, 3,5-dimethylphenyl, cyclohexylmethyl,
$R^2$ represents isopropyl, sec.-butyl, tert.-butyl, cyclopropyl, cyclohexyl, phenyl, 4-fluorophenyl, 4-hydroxyphenyl, 4-fluoro-3-methylphenyl, 3,5-dimethylphenyl, cyclohexylmethyl,
$R^3$ represents isopropyl, tert.-butyl, cyclohexyl, phenyl, 4-fluorophenyl, 4-hydroxyphenyl, 2,5-dimethylphenyl, 3,5-dimethylphenyl,
$R^4$ represents hydrogen, methyl, ethyl, sodium, potassium.

3. A compound as claimed in claim 1, wherein, in formula I or formula II,
Z represents the CH group or N,
$R^1$ represents isopropyl, cyclopropyl,
$R^2$ represents 4-fluorophenyl, 4-hydroxyphenyl,
$R^3$ represents isopropyl, tert.-butyl, cyclohexyl, phenyl, 4-fluorophenyl or 4-hydroxyphenyl, and
$R^4$ represents sodium or potassium.

4. A process for the preparation of compounds of the formulae I and II, which comprises

a) reaction of the phosphonium salts of the formula III

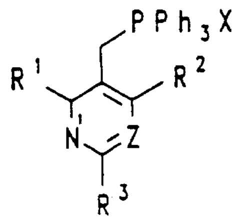

III

in which $R^1$, $R^2$, $R^3$ and Z have the meaning given for formula I, and X is Cl, Br or I, with the chiral aldehyde of the formula IV

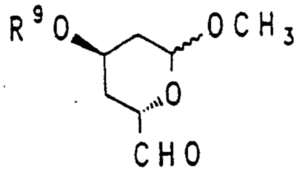

IV

in which $R^9$ is a protective group which is stable to bases and weak acids, to give a compound of the formula V

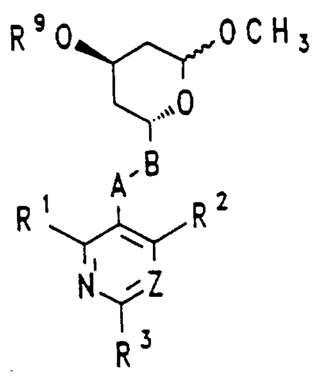

V

in which $R^1$, $R^2$, $R^3$ and Z have the meaning given for formula I, $R^9$ has the meaning given for formula IV (and A-B represents the (-CH=CH-) group),

b) acid hydrolysis of the methyl acetal group in a compound of the formula V to give a lactol of the formula VI

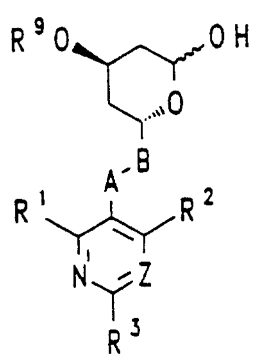

VI

in which $R^1$, $R^2$, $R^3$ and Z have the meaning given for formula I, $R^9$ has the meaning given for formula IV, and A-B represents the (-CH=CH-) group,

c) oxidation of the compound of the formula VI to give a lactone of the formula VII

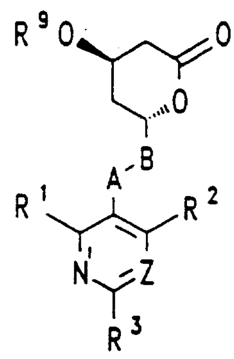

VII

in which $R^1$, $R^2$, $R^3$ and Z have the meaning given for formula I, $R^9$ has the meaning given for formula IV, and A-B represents the (-CH=CH-) group, and

d) elimination of the protective group $R^9$ in a compound of the formula VII to give a compound of the formula I in which $R^1$, $R^2$, $R^3$ and Z have the meanings given for formula I, and A-B represents the (-CH=CH-) group, and

e) where appropriate, hydrogenation of a resulting compound of the formula I, in which A-B represents a (-CH=CH-) group, to give a compound of the formula I in which A-B represents a ($-CH_2-CH_2-$) group, it also being possible for the hydrogenation to be carried out on the compounds of the formula V, VI or VII to give corresponding compounds in which A-B represents the ($-CH_2-CH_2-$) group, and

f) where appropriate, conversion of a hydroxylactone of the formula I into the corresponding dihydroxy acid II, or its salts, or, where appropriate, preparation of the corresponding esters from the hydroxylactone I or the free hydroxy acid II.

**5.** A pharmaceutical product which contains a compound as claimed in claim 1.

**6.** The use of a compound as claimed in claim 1 for the prophylaxis and therapy of arteriosclerosis and hypercholesterolemia.

**7.** A compound of the formula V

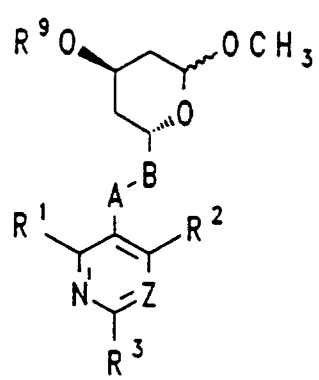

V

in which $R^1$, $R^2$, $R^3$, A-B and Z have the meaning given for formula I and $R^9$ has the meaning given for formula IV.

**8.** A compound of the formula VI

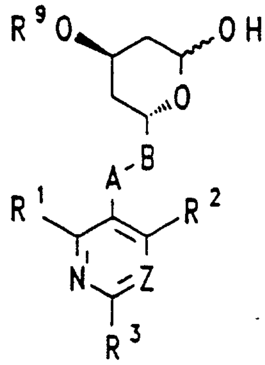

VI

in which $R^1$, $R^2$, $R^3$, A-B and Z have the meaning given for formula I and $R^9$ has the meaning given for formula IV.

**9.** A compound of the formula VII

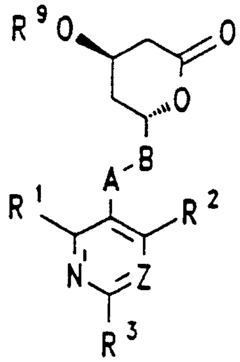

VII

in which $R^1$, $R^2$, $R^3$, A-B and Z have the meaning given for formula I and $R^9$ has the meaning given for formula IV.

**Claims for the following Contracting States : ES, GR**

**1.** A process for the preparation of a 3-demethylmevalonic acid derivative of the formula I (δ-lactone) or II (corresponding dihydroxy carboxylic acid derivative)

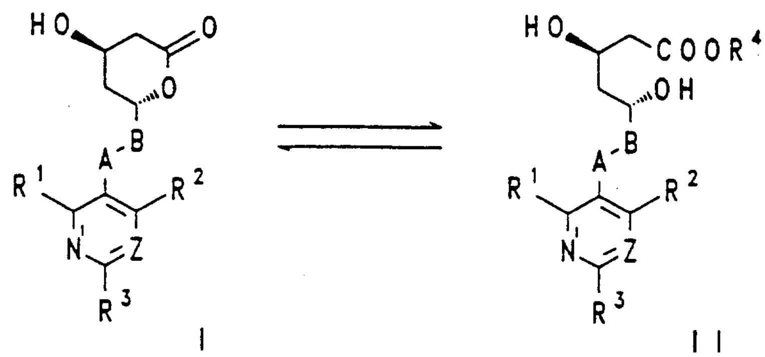

I II

in which

A-B denotes a radical of the formula -CH=CH- or $-CH_2-CH_2-$,

Z denotes a radical of the formula -CH or a nitrogen atom,

$R^1$, $R^2$ and $R^3$, independently of one another, denote a branched alkyl radical having up to 4 carbon atoms, a cycloalkyl radical having 3-6 carbon atoms or a phenyl radical which optionally carries 1 to 2 identical or different substituents selected from the group consisting of fluorine, hydroxyl and $C_1$-$C_4$-alkyl,
$R^4$ is hydrogen, methyl, ethyl, isopropyl, isobutyl, benzyl, sodium, potassium, ammonium ($NH_4$) or methyl-tris(hydroxymethyl)ammonium, which comprises

a) reaction of the phosphonium salts of the formula III

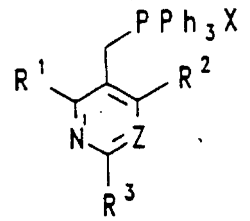

III

in which $R^1$, $R^2$, $R^3$ and Z have the meaning given for formula I, and X is Cl, Br or I, with the chiral aldehyde of the formula IV

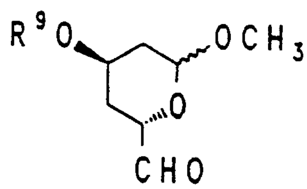

IV

in which $R^9$ is a protective group which is stable to bases and weak acids, to give a compound of the formula V

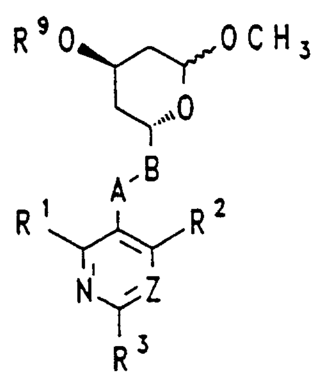

V

in which $R^1$, $R^2$, $R^3$ and Z have the meaning given for formula I, $R^9$ has the meaning given for formula IV (and A-B represents the (-CH=CH-) group),

b) acid hydrolysis of the methyl acetal group in a compound of the formula V to give a lactol of the formula VI

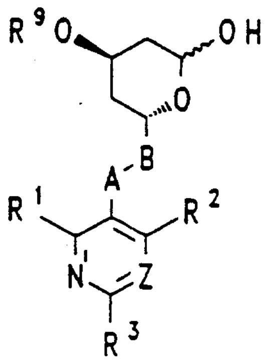

VI

in which $R^1$, $R^2$, $R^3$ and Z have the meaning given for formula I, $R^9$ has the meaning given for formula IV, and (A-B represents the (-CH=CH-) group),

c) oxidation of the compound of the formula VI to give a lactone of the formula VII

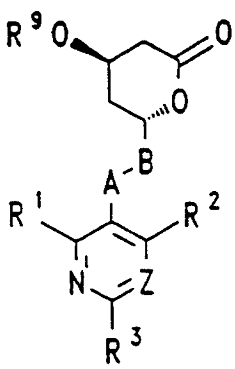

VII

in which $R^1$, $R^2$, $R^3$ and Z have the meaning given for formula I, $R^9$ has the meaning given for formula IV, and A-B represents the (-CH=CH-) group, and

d) elimination of the protective group $R^9$ in a compound of the formula VII to give a compound of the formula I in which $R^1$, $R^2$, $R^3$ and Z have the meanings given for formula I, and A-B represents the (-CH=CH-) group, and

e) where appropriate, hydrogenation of a resulting compound of the formula I, in which A-B represents a (-CH=CH-) group, to give a compound of the formula I in which A-B represents a ($-CH_2-CH_2-$) group, it also being possible for the hydrogenation to be carried out on the compounds of the formula V, VI or VII to give corresponding compounds in which A-B represents the ($-CH_2-CH_2-$) group, and

f) where appropriate, conversion of a hydroxylactone of the formula I into the corresponding dihydroxy acid II, or its salts, or, where appropriate, preparation of the corresponding esters from the hydroxylactone I or the free hydroxy acid II.

**2.** The process as claimed in claim 1, wherein a compound of the formula I or formula II is prepared in which
$R^1$ and $R^2$ denote, independently of one another, isopropyl, sec.-butyl, tert.-butyl, cyclopropyl, cyclohexyl, phenyl, 4-fluorophenyl, 4-hydroxyphenyl, 4-fluoro-3-methylphenyl, 3,5-dimethylphenyl, cyclohexylmethyl,
$R^3$ represents isopropyl, tert.-butyl, cyclohexyl, phenyl, 4-fluorophenyl, 4-hydroxyphenyl, 2,5-dimethylphenyl, 3,5-dimethylphenyl,
$R^4$ is hydrogen, methyl, ethyl, sodium, potassium.

**3.** The process as claimed in claim 1, wherein a compound of the formula I or formula II is prepared in which
$R^1$ represents isopropyl, cyclopropyl,
$R^2$ represents 4-fluorophenyl, 4-hydroxphenyl,

$R^3$ represents isopropyl, tert.-butyl, cyclohexyl, phenyl, 4-fluorophenyl or 4-hydroxyphenyl, and $R^4$ represents sodium or potassium.

4. The process as claimed in claim 1, wherein a compound of the formula V

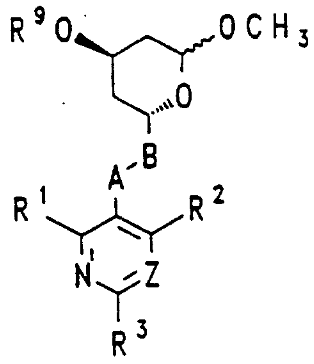

V

in which $R^1$, $R^2$, $R^3$, A-B and Z have the meaning given for formula I, $R^9$ has the meaning given for formula IV, is isolated.

5. The process as claimed in claim 1, wherein a compound of the formula VI

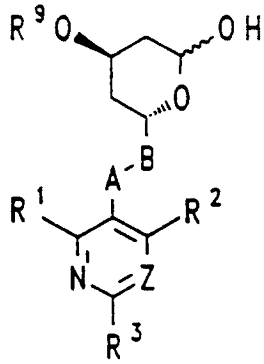

V I

in which $R^1$, $R^2$, $R^3$, A-B and Z have the meaning given for formula I, $R^9$ has the meaning given for formula IV, is isolated.

6. The process as claimed in claim 1, wherein a compound of the formula VII

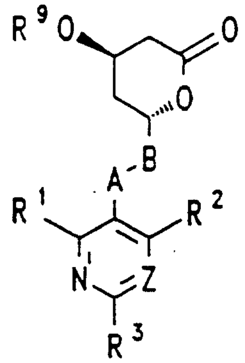

V I I

in which $R^1$, $R^2$, $R^3$, A-B and Z have the meaning given for formula I, $R^9$ has the meaning given for formula IV, is isolated.

7. The process as claimed in claim 1, wherein a compound of the formula I or II obtained is converted into a suitable administration form in a further process step.

**Revendications**

**Revendications pour les Etats contractants suivants : AT, BE, CH, DE, FR, GB, IT, LI, LU, NL, SE**

1. Dérivés d'acide 3-desméthylmévalonique de formule I (δ-lactone) ou II (dérivé d' acide dihydroxycarboxylique correspondant)

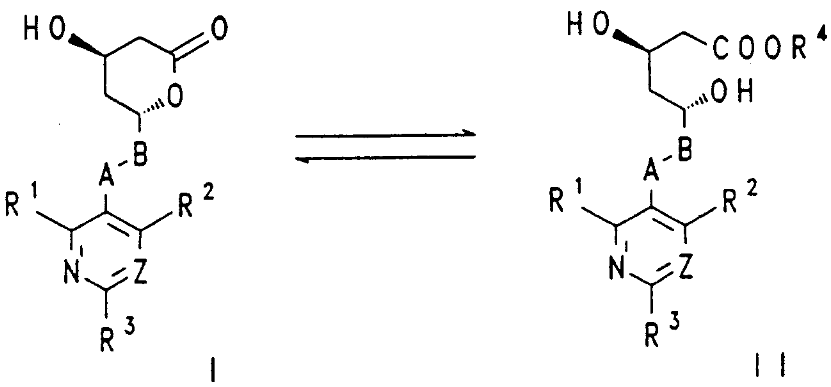

I                II

formules dans lesquelles

| | |
|---|---|
| A-B | représente un radical de formule $-CH=CH-$ ou $-CH_2-CH_2-$, |
| Z | représente un radical de formule -CH ou un atome d'azote, |
| $R^1$, $R^2$, $R^3$ | représentent, indépendamment les uns des autres, un radical alkyle ramifié ayant jusqu'à 4 atomes de carbone, un radical cycloalkyle ayant de 3 à 6 atomes de carbone ou un radical phényle qui porte éventuellement 1 ou 2 substituants, identiques ou différents, choisis parmi l'atome de fluor, le groupe hydroxy et des groupes alkyle en $C_1$-$C_4$, |
| $R^4$ | représente un atome d'hydrogène, de sodium ou de potassium, ou le groupe méthyle, éthyle, isopropyle, isobutyle ou benzyle ou l'ion ammonium ($NH_4$) ou méthyl-tris(hydroxyméthyl)ammonium. |

2. Composés selon la revendication 1, caractérisés en ce que, dans la formule I ou la formule II,

| | |
|---|---|
| $R^1$ | représente le groupe isopropyle, sec-butyle, tert-butyle, cyclopropyle, cyclohexyle, phényle, 4-fluorophényle, 4-hydroxyphényle, 4-fluoro-3-méthylphényle, 3,5-diméthylphényle, cyclohexylméthyle, |
| $R^2$ | représente le groupe isopropyle, sec-butyle, tert-butyle, cyclopropyle, cyclohexyle, phényle, 4-fluorophényle, 4-hydroxyphényle, 4-fluoro-3-méthylphényle, 3,5-diméthylphényle, cyclohexylméthyle, |
| $R^3$ | représente le groupe isopropyle, tert-butyle, cyclohexyle, phényle, 4-fluorophényle, 4-hydroxyphényle, 2,5-diméthylphényle, 3,5-diméthylphényle, |
| $R^4$ | représente un atome d'hydrogène, de sodium ou de potassium, ou le groupe méthyle ou éthyle. |

3. Composés selon la revendication 1, caractérisés en ce que, dans la formule I ou la formule II,

| | |
|---|---|
| Z | représente le groupe CH ou N, |
| $R^1$ | représente le groupe isopropyle ou cyclopropyle, |
| $R^2$ | représente le groupe 4-fluorophényle ou 4-hydroxyphényle, |
| $R^3$ | représente le groupe isopropyle, tert-butyle, cyclohexyle, phényle, 4-fluorophényle ou 4-hydroxyphényle, et |
| $R^4$ | représente un atome de sodium ou de potassium. |

4. Procédé pour la préparation de composés de formule I et de formule II, caractérisé en ce que

a) on fait réagir les sels de phosphonium de formule III

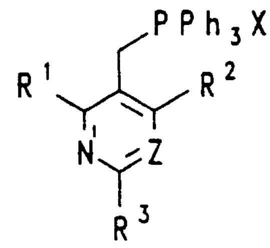

III

dans laquelle $R^1$, $R^2$, $R^3$ et Z ont les significations données à propos de la formule I, et X = Cl, Br, I, avec l'aldéhyde chiral de formule IV,

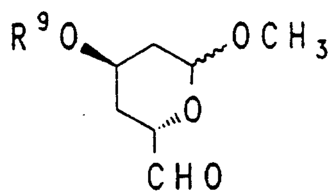

IV

dans laquelle $R^9$ représente un groupe protecteur stable en présence d'acides faibles et de bases, pour aboutir à un composé de formule V

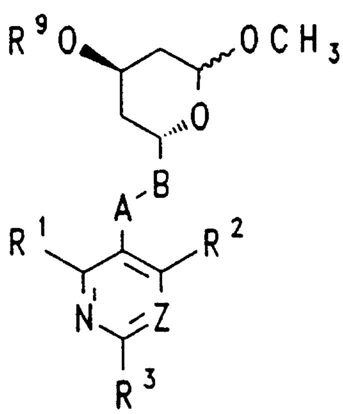

V

dans laquelle $R^1$, $R^2$, $R^3$ et Z ont les significations données à propos de la formule I et $R^9$ a la signification donnée à propos de la formule IV [et A-B représente le groupe (-CH=CH-)],

b) dans un composé de formule générale V, on soumet à une hydrolyse acide la fonction méthylacétal, pour aboutir à un lactol de formule VI,

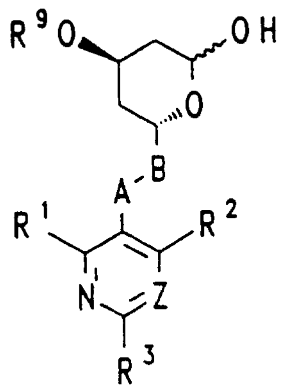

VI

dans laquelle $R^1$, $R^2$, $R^3$ et Z ont les significations indiquées à propos de la formule I, $R^9$ a la signification donnée à propos de la formule IV et A-B représente le groupe (-CH=CH-),

c) on oxyde le composé de formule générale VI en une lactone de formule VII,

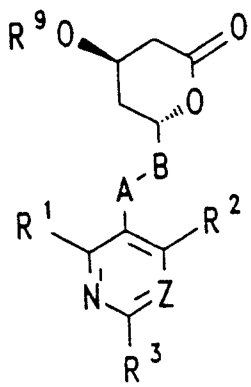

VII

dans laquelle $R^1$, $R^2$, $R^3$ et Z ont les significations données à propos de la formule I, $R^9$ a la signification donnée à propos de la formule IV, et A-B représente le groupe (-CH=CH-), et

d) dans un composé de formule générale VII, on élimine le groupe protecteur $R^9$, pour aboutir à un composé de formule I dans lequel $R^1$, $R^2$, $R^3$ et Z ont les significations données à propos de la formule I et A-B représente le groupe (-CH=CH-), et

e) éventuellement on soumet à une hydrogénation un composé de formule générale I obtenu, dans lequel A-B représente le groupe (-CH=CH-), pour aboutir à un composé de formule générale I dans lequel A-B représente le groupe ($-CH_2-CH_2-$), l'hydrogénation pouvant également s'effectuer dans le cas des composés de formule V, VI ou VII, pour aboutir aux composés correspondants dans lesquels A-B représente le groupe ($-CH_2-CH_2-$), et

f) éventuellement on convertit une hydroxylactone de formule I en l'acide dihydroxycarboxylique II correspondant ou ses sels ou éventuellement, on prépare les esters correspondants à partir de l'hydroxylactone I ou de l'acide hydroxycarboxylique libre II.

**5.** Composition pharmaceutique, caractérisée en ce qu'elle contient un composé selon la revendication 1.

**6.** Utilisation de composés selon la revendication 1, pour la prophylaxie et le traitement de l'artériosclérose et de l'hypercholestérolémie.

**7.** Composés de formule V

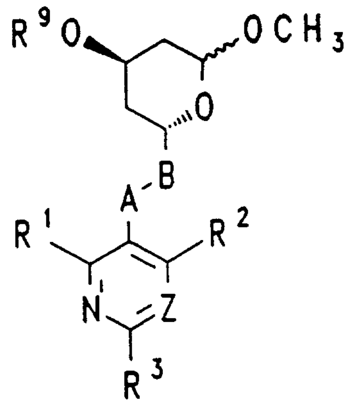

V

dans laquelle $R^1$, $R^2$, $R^3$, A-B et Z ont les significations données à propos de la formule I, et $R^9$ a la signification donnée à propos de la formule IV.

**8.** Composés de formule VI

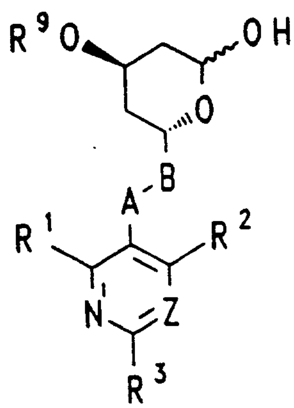

VI

dans laquelle $R^1$, $R^2$, $R^3$, A-B et Z ont les siqnifications données à propos de la formule I, et $R^9$ a la signification donnée à propos de la formule IV.

**9.** Composés de formule VII

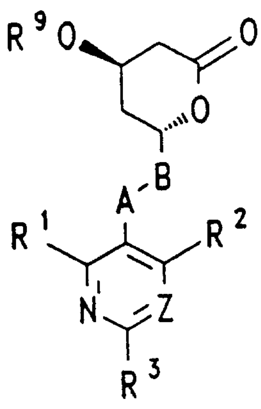

VII

dans laquelle $R^1$, $R^2$, $R^3$, A-B et Z ont les significations données à propos de la formule I, et $R^9$ a la signification donnée à propos de la formule IV.

**Revendications pour les Etats contractants suivants : ES, GR**

**1.** Procédé pour la préparation de dérivés d'acide 3-desméthylmévalonique de formule I (δ-lactone) ou II (dérivé d' acide dihydroxycarboxylique correspondant)

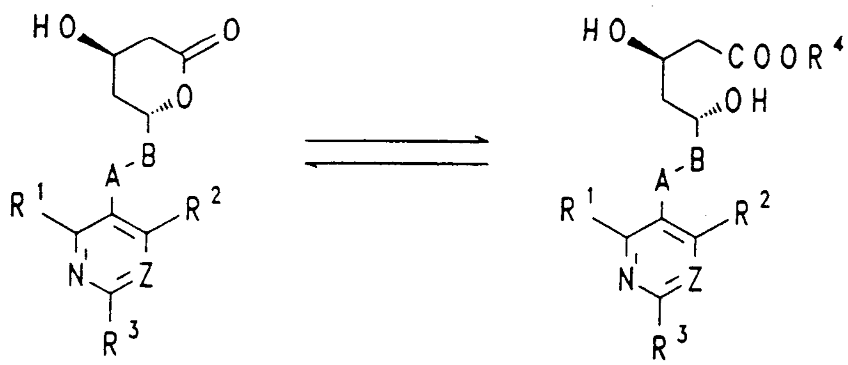

I II

formules dans lesquelles

| | |
|---|---|
| A-B | représente un radical de formule $-CH=CH-$ ou $-CH_2-CH_2-$, |
| Z | représente un radical de formule -CH ou un atome d'azote, |
| $R^1$, $R^2$, $R^3$ | représentent, indépendamment les uns des autres, un radical alkyle ramifié ayant jusqu'à 4 atomes de carbone, un radical cycloalkyle ayant de 3 à 6 atomes de carbone ou un radical phényle qui porte éventuellement 1 ou 2 substituants, identiques ou différents, choisis parmi l'atome de fluor, le groupe hydroxy et des groupes alkyle en $C_1$-$C_4$, |
| $R^4$ | représente un atome d'hydrogène, de sodium ou de potassium, ou le groupe méthyle, éthyle, isopropyle, isobutyle ou benzyle ou l'ion ammonium ($NH_4$) ou méthyl-tris(hydroxyméthyl)ammonium, |

caractérisé en ce que

a) on fait réagir les sels de phosphonium de formule III

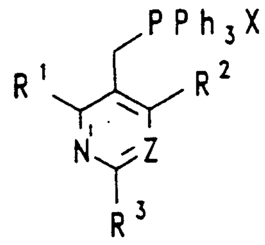

III

dans laquelle $R^1$, $R^2$, $R^3$ et Z ont les significations données à propos de la formule I, et X = Cl, Br, I, avec l'aldéhyde chiral de formule IV,

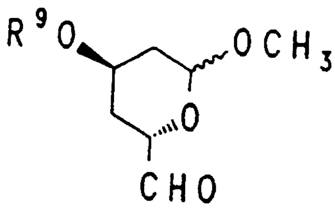

IV

dans laquelle $R^9$ représente un groupe protecteur stable en présence d'acides faibles et de bases, pour aboutir à un composé de formule V

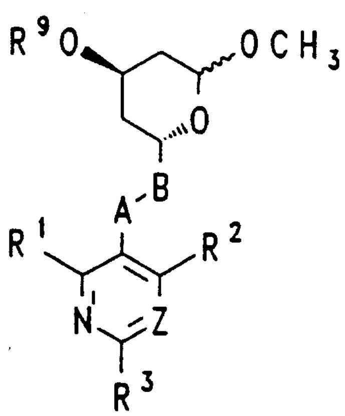

V

dans laquelle $R^1$, $R^2$, $R^3$ et Z ont les significations données à propos de la formule I et $R^9$ a la signification donnée à propos de la formule IV [et A-B représente le groupe (-CH=CH-)],

b) dans un composé de formule générale V, on soumet à une hydrolyse acide la fonction méthylacétal, pour aboutir à un lactol de formule VI,

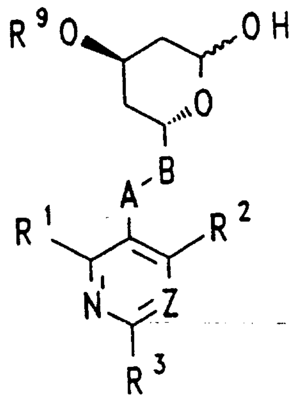

VI

dans laquelle $R^1$, $R^2$, $R^3$ et Z ont les significations indiquées à propos de la formule I, $R^9$ a la signification donnée à propos de la formule IV et A-B représente le groupe (-CH=CH-),

c) on oxyde le composé de formule générale VI en une lactone de formule VII,

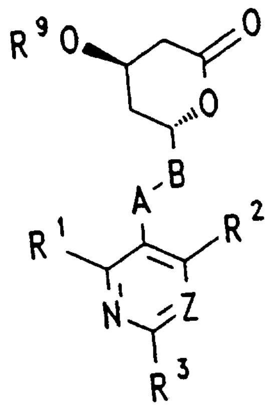

VII

dans laquelle $R^1$, $R^2$, $R^3$ et Z ont les significations données à propos de la formule I, $R^9$ a la signification donnée à propos de la formule IV, et A-B représente le groupe (-CH=CH-), et

d) dans un composé de formule générale VII, on élimine le groupe protecteur $R^9$, pour aboutir à un composé de formule I dans lequel $R^1$, $R^2$, $R^3$ et Z ont les significations données à propos de la formule I et A-B représente le groupe (-CH=CH-), et

e) éventuellement on soumet à une hydrogénation un composé de formule générale I obtenu, dans lequel A-B représente le groupe (-CH=CH-), pour aboutir à un composé de formule générale I dans lequel A-B représente le groupe ($-CH_2-CH_2-$), l'hydrogénation pouvant également s'effectuer dans le cas des composés de formule V, VI ou VII, pour aboutir aux composés correspondants dans lesquels A-B représente le groupe ($-CH_2-CH_2-$), et

f) éventuellement on convertit une hydroxylactone de formule I en l'acide dihydroxycarboxylique II correspondant ou ses sels ou éventuellement, à partir de l'hydroxylactone I ou de l'acide hydroxycarboxylique libre II, on prépare les esters correspondants.

2. Procédé selon la revendication 1, caractérisé en en ce que l'on prépare un composé de formule I ou de formule II dans lequel

$R^1$ et $R^2$ représentent, indépendamment l'un de l'autre, le groupe isopropyle, sec-butyle, tert-butyle, cyclopropyle, cyclohexyle, phényle, 4-fluorophényle, 4-hydroxyphényle, 4-fluoro-3-méthylphényle, 3,5-diméthylphényle, cyclohexylméthyle,

$R^3$ représente le groupe isopropyle, tert-butyle, cyclohexyle, phényle, 4-fluorophényle, 4-hydroxyphényle, 2,5-diméthylphényle, 3,5-diméthylphényle,

$R^4$ représente un atome d'hydrogène, de sodium ou de potassium, ou le groupe méthyle ou éthyle.

3. Procédé selon la revendication 1, caractérisé en ce que l'on prépare un composé de formule I ou de formule II dans lequel

$R^1$ représente le groupe isopropyle ou cyclopropyle,
$R^2$ représente le groupe 4-fluorophényle ou 4-hydroxyphényle,
$R^3$ représente le groupe isopropyle, tert-butyle, cyclohexyle, phényle, 4-fluorophényle ou 4-hydroxyphényle, et
$R^4$ représente un atome de sodium ou de potassium.

4. Procédé selon la revendication 1, caractérisé en ce que l'on isole des composés de formule V

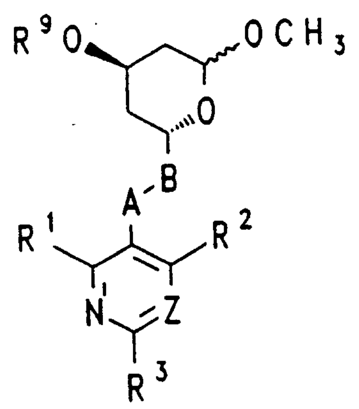

V

dans laquelle $R^1$, $R^2$, $R^3$, A-B et Z ont les significations données à propos de la formule I, et $R^9$ a la signification donnée à propos de la formule IV.

5. Procédé selon la revendication 1, caractérisé en ce que l'on isole des composés de formule VI

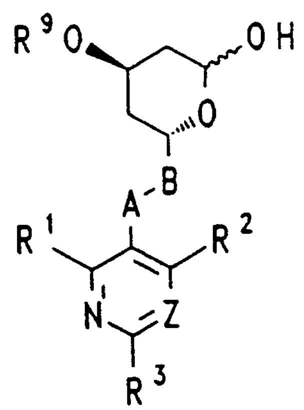

VI

dans laquelle $R^1$, $R^2$, $R^3$, A-B et Z ont les significations données à propos de la formule I, et $R^9$ a la signification donnée à propos de la formule IV.

6. Procédé selon la revendication 1, caractérisé en ce que l'on isole des composés de formule VII

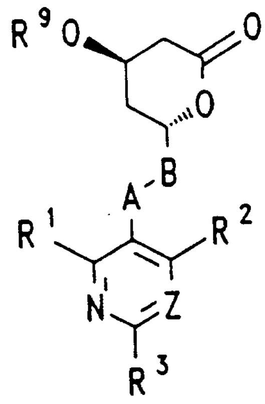

VII

dans laquelle $R^1$, $R^2$, $R^3$, A-B et Z ont les significations données à propos de la formule I, et $R^9$ a la signification donnée à propos de la formule IV.

7. Procédé selon la revendication 1, caractérisé en ce que, dans une autre étape du procédé, on met sous une forme d'administration appropriée un composé de formule I ou II obtenu.